# EUROPÄISCHE PATENTSCHRIFT

(11) **EP 2 059 525 B1**
(45) Veröffentlichungstag und Bekanntmachung des Hinweises auf die Patenterteilung: **21.12.2011**
(21) Anmeldenummer: 07786022.9
(22) Anmeldetag: 12.07.2007
(51) Int. Cl.: C07K 14/02

(54) **SPLIT-CORE-PARTIKEL FÜR DIE PRÄSENTATION VON FREMDMOLEKÜLEN, INSBESONDERE FÜR IMPFSTOFFARTWENDUNGEN UND VERFAHREN ZU DEREN HERSTELLUNG**
SPLIT-CORE-PARTICLES FOR THE PRESENTATION OF FOREIGN MOLECULES, ESPECIALLY FOR VACCINE APPLICATIONS, AND METHOD FOR THEIR PRODUCTION
PARTICULES À NOYAU FENDU POUR LA PRÉSENTATION DE MOLÉCULES ÉTRANGÈRES, NOTAMMENT POUR VACCINS ET LEUR PROCÉDÉ DE FABRICATION

(30) Priorität: 08.09.2006 EP 06018847
(43) Veröffentlichungstag der Anmeldung: 20.05.2009
(73) Patentinhaber: Universitätsklinikum Freiburg, 79106 Freiburg (DE)
(72) Erfinder: NASSAL, Michael, 79232 March (DE); SKAMEL, Claudia, 79108 Freiburg (DE); WALKER, Andreas, 45357 Essen (DE)
(74) Vertreter: Keller, Günter
(86) Internationale Anmeldenummer: PCT/EP2007/006190
(87) Internationale Veröffentlichungsnummer: WO 2008/028535

(56) Entgegenhaltungen:
- US-A1- 2003 175 863
- VOGEL MAREN ET AL: "Quaternary structure is critical for protein display on capsid-like particles (CLPs): Efficient generation of hepatitis B virus CLPs presenting monomeric but not dimeric and tetrameric fluorescent proteins" PROTEINS STRUCTURE FUNCTION AND BIOINFORMATICS, Bd. 58, Nr. 2, 1. Februar 2005 (2005-02-01), Seiten 478-488, XP009078277
- VOGEL ET AL: "In vitro assembly of mosaic hepatitis B virus capsid-like particles (CLPs): Rescue into CLPs of assembly-deficient core protein fusions and FRET-suited CLPs" FEBS LETTERS, ELSEVIER, AMSTERDAM, NL, Bd. 579, Nr. 23, 26. September 2005 (2005-09-26), Seiten 5211-5216, XP005390469 ISSN: 0014-5793
- SKAMEL CLAUDIA ET AL: "Hepatitis B virus capsid-like particles can display the complete, dimeric outer surface protein C and stimulate production of protective antibody responses against Borrelia burgdorferi infection" JOURNAL OF BIOLOGICAL CHEMISTRY, Bd. 281, Nr. 25, Juni 2006 (2006-06), Seiten 17474-17481, XP009078268 ISSN: 0021-9258 in der Anmeldung erwähnt
- O'SHEA E K ET AL: "PEPTIDE 'VELCRO*': DESIGN OF A HETERODIMERIC COILED COIL" CURRENT BIOLOGY, CURRENT SCIENCE,, GB, Bd. 3, Nr. 10, 1993, Seiten 658-667, XP000653001 ISSN: 0960-9822 in der Anmeldung erwähnt

## Beschreibung

Gegenstand der vorliegenden Erfindung sind neue Träger für Impfstoffe, aber auch für andere Moleküle, auf der Grundlage des Core-Antigens des Hepatitis B-Virus. In das Core-Antigen des Hepatitis-Virus werden fremde Aminosäuresequenzen eingebaut, die von Krankheitserregern stammen können. Gegen diese Proteinsequenzen sollen durch den Impfstoff Antikörper gebildet werden, wobei schützende oder neutralisierende Antikörper bevorzugt sind. Durch die erfindungsgemäßen Partikel kann auch die zelluläre Immunantwort (T-Zellen) stimuliert werden. Das Core-Protein von Hepatitis B hat die Eigenschaft, dass sich mehrere Kopien zu sogenannten capsid-like particles zusammenfügen können. Diese capsid-like particles (CLP) eignen sich besonders für die Herstellung von Impfstoffen, de sie das Immunsystem stimulieren und so zu einer gesteigerten Antikörperproduktion führen. Der Ausdruck "Impfstoff" ist in der vorliegenden Anmeldung bevorzugt als Trägersystem zu verstehen, das sowohl humorale wie auch zelluläre Immunantworten hervorrufen kann.

Kapside von Hepatitis B Viren sind ikosahedral symmetrische Nanopartikel (Durchmesser ca. 30 nm), die aus 180 bzw. 240 Kopien des viralen Core-Proteins bestehen. Das Core-Protein wird als HBcAg bezeichnet. Sie können als partikulärer Träger für Fremdmoleküle dienen; die bevorzugte Anwendung ist die als immunverstärkender Antigen-Träger für Impfstoffe. Durch geeignete Modifikation des Core-Proteins können die Fremdmoleküle auf der Partikel-Oberfläche (bei Bedarf auch im Inneren) präsentiert werden. Für die Oberflächenexposition ist die Verknüpfung des Fremdmoleküls mit zentral in der AS-Sequenz des Core-Proteins gelegenen AS-Resten (Bereich der AS ca. 73-94) optimal, welche das immundominante B-Zellepitop "c/e1" enthalten und in der 3D-Struktur am stärksten auf der Partikeloberfläche exponiert sind (AS bedeutet Aminosäure).

Die WO 01/77158 beschreibt Hepatitis B-Core-Antigen-Fusionsproteine, wobei heterologe Epitope bevorzugt in den Bereich zwischen den Positionen 61 und 90 eingesetzt werden. Die US 2003/0198649 beschreibt Hepatitis B-Virus Core-Antigen-Partikel, bei der die Immunogene über Ligandenstrukturen mit dem Hepatitis B-Virus (HBV) Core-Protein verbunden sind. Kratz et al. [PNAS (1999), 1915-1920] beschreiben die Präsentation von GFP (Green Fluorescent Protein) auf der Oberfläche des Coreproteins von HBV. Dabei wurden die Aminosäuren 79 und 80 des Core-Proteins durch die 238 Aminosäure lange Sequenz von GFP ersetzt.

Nassal et al. [Eur.J.Immunol. (2005), S. 655-665] beschreiben ein Fusionsprodukt des gesamten OspA-Proteins von Borrelia burgdorferi und des Hepatitis B-Virus-Kapsidproteins. Auch hier wurden die Aminosäuren 79 und 80 durch die Aminosäuren 18 bis 273 von OspA ersetzt, wobei allerdings zwischen Core-Protein und OspA Linkersequenzen eingebaut wurden. Skamel et al. (Journal of Biological Chemistry, 2006, Seiten 17474-17481) beschreiben die Präsentation des kompletten OspC-Proteins von Borrelia burgdorferi mit Hilfe von Hepatitis B-Virus-Kapsid-ähnlichen Partikeln. Bei den aus dem Stand der Technik bekannten Lösungen hat sich aber herausgestellt, dass die veränderten Coreproteine nicht mehr in gewünschter Weise zur Bildung von Kapsid-ähnlichen Partikeln (CLPs) neigen und, dass daher die Immunantwort nicht mehr zufriedenstellend stimuliert wird.

Bei einer genetischen Insertionen von Fremdmolekülen auf Peptid- bzw. Proteinbasis in das Core-Protein bedeutet dies, dass die Fremdsequenz sowohl über ihren N- wie ihren C-Terminus mit dem Core-Protein verbunden sind. Es wurde nun gefunden, dass die beidseitige Verknüpfung die Art und Auswahl geeigneter Fremdsequenzen drastisch einschränkt.

Das hier beschriebene "split-Core-System" hebt diese Einschränkungen auf, indem das Core-Protein in zwei separaten Teilen erzeugt wird, die sich überraschenderweise spontan zusammenfinden und vergleichbar der kontinuierlichen Proteinkette Kapsidpartikel bilden.

Fremdmoleküle können entweder an das N-terminale ("coreN") oder das C-terminale ("coreC") Fragment fusioniert werden und sind dadurch nur noch über *ein* Ende mit dem Trägerprotein verknüpft. Die strukturellen Einschränkungen der beidseitigen Verknüpfung bei der Insertion von Fremdsequenzen in die kontinuierliche Peptidkette des Core-Proteins fallen damit weitestgehend weg.

Das erfindungsgemäße Split-Core-System erlaubt daher:
(i) Die Präsentation von Fremdmolekülen, die aufgrund ihrer Größe und/oder Struktur im konventionellen kontinuierlichen Core-Protein-Kontext nicht, oder nur sehr schlecht, präsentiert werden können;
(ii) Die Präsentation von heterodimeren Fremdproteinen;
(iii) Die Präsentation von interaktionsfähigen Fremdmolekülen in einer flexiblen, gut zugänglichen Form, welche die Interaktion mit den gewünschten Partnermolekülen wesentlich erleichtert;
(iv) Die Präsentation zusätzlicher Fremdmoleküle über die im split-Core, nicht aber im herkömmlichen kontinuierlichen Core-System, vorhandenen zusätzlichen oberflächenexponierten N- und C-Termini.

Hepatitis B Viren (HBVs) sind umhüllte Viren. Das innere Nukleokapsid wird auch als Corepartikel ("core") bezeichnet; serologisch ist es als Hepatitis B core antigen (HBcAg) definiert. Cores bestehen aus 180 bzw. 240 Kopien des 183-185 AS langen Core-Proteins (abhängig vom HBV Subtyp). Das Core-Protein kann heterolog (in Bakterien, Hefe, eukaryonten Zellen) exprimiert werden; dabei bilden sich spontan "capsid-like particles" (=CLPs). Da sie weder das Virusgenom noch die äußere Hülle enthalten, sind sie nicht infektiös. Solche CLPs lassen sich auch von den näher verwandten Säuger-HBVs (z.B. Waldmurmeltier = woodchuck; Erdhörnchen = grounds squirrel; und weitere) sowie den entfernter verwandten Vogel-HBVs (z.B. Ente = duck; Reiher = heron; und weitere) erhalten. Grundsätzlich können erfindungsgemäß alle HBV Sequenzen eingesetzt werden, bevorzugt sind allerdings die Sequenzen von HBVs, die Menschen infizieren können.

Die Aminosäuresequenz bzw. Nucleotidsequenz des HBV-Core-Proteins ist bekannt [Galibert et al., Nature (1979), S. 646-650; Nassal, Gene (1988), Seiten 279-294 oder WO 01/77158], auf diese Veröffentlichungen wird Bezug genommen. Bevorzugt eingesetzt wird die Core-Protein-Sequenz des Subtyps ayw, es können aber auch Varianten, Modifikationen der HBV-Sequenz ebenso verwendet werden, wie die Sequenz von anderen Säuger-HBVs oder Vogel-HBVs. Die Sequenzen sind in den öffentlich zugänglichen Genbanken gespeichert.

Neben dem humanpathogenen Hepatitis B Virus (HBV im engen Sinn) gibt es eine Reihe verwandter, tier-spezifischer Hepatitis B Viren, z.B. das HBV des Nordamerikanischen Waldmurmeltiers (Englisch: woodchuck; zool. *Marmota monax;* woodchuck hepatitis B virus = WHV), das HBV des kalifornischen Erdhörnchens (Englisch: California ground squirrel; zool. *Spermophilus beecheyi;* ground squirrel hepatitis B virus = GSHV), sowie weitere.

Diese Viren besitzen eine ähnliche genetische Struktur wie das humane HBV, weisen aber distinkte Unterschiede in ihrer Nukleotid- und damit Aminosäure-Sequenz ihrer Proteine auf.

Die Verwendung von humanem HBV Core als Impfstoff-Träger unterliegt zwei möglichen Einschränkungen:
1. Personen mit einer chronischen HBV-Infektion sind partiell tolerant gegen HBV-Antigene (diese fehlende Immunantwort ist mitverantwortlich für die Persistenz der Infektion). Core wirkt auch als T-Zell-abhängiges Antigen. T-Zell-Epitope in der Sequenz des Coreproteins sind daher an der starken Immunogenität von Impfstoffen auf HBV Core-CLP-Basis beteiligt. In chronisch HBV-Infizierten würde dies möglicherweise aufgrund ihrer T-Zell-Toleranz gegen HBV Core nicht zum Tragen kommen. Eine solche Toleranz besteht jedoch nicht gegen Core von WHV und anderen Tier-HBVs (vgl. Billaud JN, et al. Advantages to the use of rodent hepadnavirus core proteins as vaccine platforms. Vaccine. 2007 Feb 19;25(9):1593-606). Für diesen speziellen Kreis von potentiellen Impflingen könnte daher die Verwendung eines Tier-HBV Coreproteins als Träger von Vorteil sein.
2. Aufgrund seiner außergewöhnlich starken Immunogenität ruft Core bei allen HBV-Infizierten, ob akut oder chronisch, eine starke anti-Core B-Zellantwort hervor (anti-HBcAg); ausgeheilte Infektionen sind zusätzlich durch das Auftreten von Antikörpern gegen die HBV Hüllproteine (anti-HBsAg) charakterisiert. Derzeitige prophylaktische Impfstoffe gegen die HBV-Infektion basieren ausschließlich auf dem HBV Hüllprotein (HBsAg). Erfolgreiche Impfung bewirkt daher - so wie überstandende Infektion - das Auftreten von anti-HBsAg. In einigen Fällen ist es von diagnostischem Interesse, ob eine Person mit anti-HBsAg dies durch Infektion, oder durch HBsAg-Impfung, erhalten hat - dies lässt sich durch den Nachweis von anti-HBcAg unterscheiden, welches nur nach Infektion auftritt.

Ein Impfstoff auf Basis von humanem HBV-Core würde neben der erwünschten Antwort gegen das inserierte Fremdprotein auch eine gewisse anti-Core (anti-HBcAg)-Antwort hervorrufen. Dies würde bei gleichzeitiger Anwesenheit von anti-HBsAg die Unterscheidung HBV-Infektion vs. HBsAg-Impfung erschweren.

Im split-Core-System ist die Entstehung von anti-HBcAg bereits minimiert, da das Hauptepitop, nämlich c/e1, physikalisch getrennt vorliegt und somit von der überwiegenden Anzahl von anti-HBcAg Antikörpern nicht mehr erkennbar ist. Eine Rest-anti-HBcAg Antwort gegen andere Sequenzbereiche des Coreproteins ist jedoch vorhanden. Dies lässt sich durch Verwendung eines Nicht-human-HBV-Coreproteins, bevorzugt des WHV-Coreproteins, als Trägerbasis verhindern bzw weiter vermindern.

Der wesentliche Bestandteil des erfindungsgemäßen Split-Core-Trägersystems ist einerseits das Core-Protein eines Hepatitis B-Virus und andererseits das Fremdmolekül, gegen das eine Immunantwort hervorgerufen werden soll.

Das Core-Protein wird in der breitesten Ausführungsform von einem beliebigen Hepatitis B-Virus abgeleitet. Es sind verschiedene Hepatitis B-Viren bekannt. Bevorzugt eingesetzt werden erfindungsgemäß solche Sequenzen, die von Hepatitis B-Viren abgeleitet sind, die aus Säugetieren isoliert wurden und für diese spezifisch sind Besonders bevorzugt wird humanes HBV eingesetzt. Daneben können aber auch solche Hepatitis B-Viren die Sequenz des Core-Proteins liefern, die von anderen Tieren, beispielsweise Vögeln, wie Enten oder Reihern, herstammen.

Die andere Komponente, nämlich das Fremdmolekül, gegen das eine Immunantwort hervorgerufen werden soll, kann prinzipiell jedes beliebige Molekül sein. Bevorzugt handelt es sich hierbei um Proteinsequenzen und besonders bevorzugt um solche Proteinsequenzen, die auf den Oberflächen von Krankheitserregern gefunden werden und, die mit dem Immunsystem in Kontakt treten. Wenn das Immunsystem Antikörper gegen derartige Oberflächenstrukturen von Krankheitserregern ausbilden kann, werden die Krankheitserreger nach Kontakt mit den einzelnen Komponenten des Immunsystem des geimpften Patienten inaktiviert, wobei der eingedrungene Krankheitserreger in der Regel zerstört wird.

Wenn man die in Datenbanken verfügbaren Sequenzen des woodchuck Hepatitis B-Virus (WHV) mit der Aminosäure des Coreproteins von HBV (Subtyp ayw) vergleicht, stellt man fest, dass eine Identität von etwa 60 % vorliegt. Die Sequenzunterschiede sind im Bereich der Aminosäurepositionen zwischen 66 und 94 besonders ausgeprägt, wobei diese Sequenz das c/e1-Epitop und im erfindungsgemäßen HBV-Split-Core-System die Trennstelle zwischen N-terminalem Bereich und C-terminalem Bereich des Coreproteins enthält. Diese Trennstelle liegt in einer besonders bevorzugten Ausführungsform zwischen Aminosäure 79 und 81.

Für die Herstellung von erfindungsgemäßen WHV-Split-Core-Trägersystemen wurde die Coreprotein-Sequenz des WHV-Coreproteins zwischen der Position 79 (kodiert für Aminosäure Glu) und der Position 80 (kodiert für die Aminosäure Glutamin) auf Nucleinsäure-Ebene getrennt. Auf Nucleotidebene wurde an den Carboxyterminus des N-terminalen Coreprotein-Segments von WHV (kurz als WcoreN bezeichnet) eine Sequenz kodierende BamHI-Schnittstelte angeführt. Daran schloß sich eine zweite Ribosomenbindungsstelle sowie eine Schnittstelle für die Restriktionsendonuclease Nde1 an, welche wiederum mit dem Startkodon des WHV coreC (WcoreC)-Segmentes überlappt. Diese Anordnung ist identisch mit der bevorzugten Anordnung in den entsprechenden HBV-Split-Core-Konstrukten. Somit können Insertionen direkt mit Hilfe eines BamHI-Nde1-Verdaus von einem in das andere System transferiert werden. Im vorliegend beschriebenen Fall enthält WcoreN durch die eingeführte BamHI-Schnittstelle einen konservativen Austausch, nämlich E79D, und zusätzlich ein P als C-terminale Aminosäure. Dies ist unschädlich für die Partikelbildungsfähigkeit und das C-terminale Prolin, erhöht aufgrund seiner Protease-Resistenz die Stabilität des Split-Proteins. Analog HBV Split-Core enthält WcoreC ein zusätzliches Start-Mathionin, in diesem Fall vor Q80, bei HBV vor 381. Dieses WHV-Split-Core-Konstrukt bildete ebenso gut Partikel wie das entsprechende HBV-Split-Core-Konstrukt.

In einer weiteren Ausführungsform der vorliegenden Erfindung können auch der C-terminale Bereich und der N-terminale Bereich des Coreproteins von unterschiedlichen Hepatitis B-Viren herstammen. Das erfindungsgemäße Hybrid-Split-Core-Trägersystem weist in diesem Fall im wesentlichen drei Komponenten auf, den N-terminalen Bereich des Coreproteins von einem Hepatitis B-Virus, beispielsweise einem humanen Hepatitis B-Virus, das Fremdmolekül, gegen das eine Immunantwort hervorgerufen werden kann und den C-terminalen Bereich des Coreproteins, das von einem anderen Hepatitis B-Virus herstammt, beispielsweise von dem WHV. Auch von einem solchen Hybrid-Split-Core-Konstrukt wurden problemlos Partikel gebildet. Da sich die Aminosäure-Sequenzen der Coreproteine aus unterschiedlichen Hepatitis B-Viren unterscheiden, unterscheiden sich auch die B- und T-Zellepitope. Somit kann durch das Hybrid-Split-Core-System die Immunantwort gegen den partikulären Träger gezielt beeinflusst werden.

Für die Herstellung von bevorzugten erfindungsgemäßen Hybrid-Split-Core-Trägersystemen wurde die Coreprotein-Sequenz des WHV-Coreproteins zwischen der Position 79 (kodiert für Aminosäure Glu) und der Position 80 (kodiert für die Aminosäure Glutamin) auf Nucleinsäure-Ebene getrennt. Auf Nucleotidebene wurde an den Carboxyterminus des N-terminalen Coreproteins von WHV (kurz als W-Core-N bezeichnet) eine Sequenz kodierende BamHI-Schnittstelle angeführt. Daran schloß sich eine zweite Ribosomenbindungsstelle sowie eine Schnittstelle für die Restriktionsendonuclease Nde1 an, welche wiederum mit dem Startkodon des WHV coreC (WcoreC)-Segmentes überlappt. Diese Anordnung ist identisch mit der bevorzugten Anordnung in den entsprechenden HBV-Split-Core-Konstrukten. Somit können Insertionen direkt mit Hilfe eines BamHI-Nde1-Verdaus von einem in das andere System transferiert werden. Im vorliegend beschriebenen Fall enthält WcoreN durch die eingeführte BamHI-Schnittstelle einen konservativen Austausch, nämlich E79D, und zusätzlich ein P als C-terminale Aminosäure. Dies ist unschädlich für die Partikelbildungsfähigkeit und das C-terminale Prolin, erhöht aufgrund seiner Protease-Resistenz die Stabilität des Split-Proteins. Analog HBV Split-Core enthält WcoreC ein zusätzliches Start-Methionin, in diesem Fall vor Q80, bei HBV vor S81. Bei diesem Konstrukt konnte Partikel problemlos gebildet werden.

Natürliches HBcAg wie auch rekombinante HBV CLPs sind hochimmunogen (T-Zellunabhängig und T Zell-abhängiges Antigen). Wesentlich dafür ist die symmetrische, multimere partikuläre Struktur des Corepartikels. Diese lässt sich in konventioneller "negative stain" Elektronenmikroskopie und auch Kryo-Elektronenmikroskopie darstellen. Biochemisch lässt sich die partikuläre Natur nachweisen durch Sedimentation in Sucrosegradienten (Sedimentationskoeffizient je nach Variante 60 - 80 S; zum Vergleich: lösliche monomere Proteine ca. 1-5 S; eukaryonte ribosomale Untereinheiten 40S+ 60S). Außerdem haben die Partikel ein distinktes Laufverhalten bei der nativen Gelelektrophorese in Agarosegelen, das sich von den nicht-partikulärer Formen unterscheidet. Dadurch ist feststellbar, ob sich CLP bilden können, oder ob die Proteine ohne die CLP-Struktur vorliegen.

Das Core-Protein von HBV weist 183 oder 185 Aminosäuren (das von WHV 187 Aminosäuren) auf und besteht aus zwei Domänen: die ersten ca. 140 AS sind notwendig und hinreichend für das Assembly zu CLPs ("Assemblydomäne"); die C-terminale Region ist reich an basischen AS und bindet Nukleinsäuren. Eine C-terminal verkürzte Core-Protein-Variante (AS1-149) wurde in den ursprünglichen E. coli Expressionssystemen wesentlich besser exprimiert als das Volllängenprotein. CLPs aus Core-Protein 1-149, d.h. ohne Nukleinsäure-Bindungsdomäne wurden daher für mittel- und hochauflösende EM-Strukturuntersuchungen benutzt. So konnte die Kristallstruktur der CLPs und des Proteins bestimmt werden. Wie biochemisch vorhergesagt, bildet das Core-Protein sehr stabile, symmetrische Dimere, die sich zu CLPs aus 90 oder 120 Dimeren zusammenlagern.

Die Corepartikel besitzen auffällige "spikes"; jedes Dimer bildet einen spike, der strukturell aus einem 4-Helixbündel besteht, zu dem jedes Monomer 2 lange, zentral in der AS-Sequenz gelegene a-Helices beiträgt. Die spike-Spitzen umfassen die Aminosäuren (AS) in der Region von AS 74-85. Diese Region trägt auch das immundominante B-Zell-Epitop des HBcAg (c/e1-Epitop), seine besonders hohe Immunogenität erklärt sich aus der Oberflächen-Exposition dieser Reste.

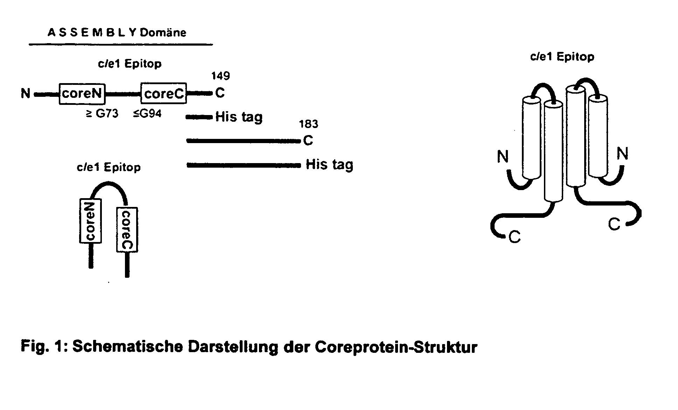

Figur 1 zeigt die Core-Protein-Struktur schematisch. Auf der linken Seite der Figur 1 ist die Primärsequenz des HBV-Core-Proteins dargestellt. Das Gesamtprotein besteht aus 183 AS (in einigen HBV Subtypen 185 AS); nur die N-terminalen ca. 140 AS werden zur CLP-Bildung benötigt (Assembly-Domäne); eine bevorzugte C-terminale AS-Position ist 149; an diese und jede folgende AS, bis AS183, kann ein His-tag fusioniert werden, ohne die CLP zu stören. Das c/e1-Epitop liegt im Bereich der AS 78 - 83, und bildet einen Teil des Loops zwischen den zentralen α-Helices (links unten). Die Helices selbst können verkürzt werden; minimal benötigt werden wahrscheinlich in coreN die AS-Reste bis Pos. Gly73, in coreC die Reste ab AS Gly94. Die rechte Seite der Fig. 1 zeigt eine schematische Darstellung eines Core-Protein-Dimers. Nur die beiden zentralen Helices pro Monomer sind gezeigt. Das c/e1 Epitop ist auf der Partikeloberfläche.

Für eine erfolgreiche Partikelbildung muss die Core AS-Sequenz mindestens Pos. 140 enthalten, vorzugsweise mindestens Pos. 149, kann sich aber auch weiter erstrecken bis zur C-terminalen AS 183 bzw. 185, je nach HBV Subtyp. Die AS-Sequenz nach Pos. 140, besser 149, kann durch Fremdsequenzen ersetzt werden, diese Fremdsequenzen befinden sich dann üblicherweise im Inneren der CLPs. Gegen solche Fremdaminosäuren werden nicht in erster Linie Antikörper gebildet, sie können aber andere Funktionen erfüllen.

Aufgrund der außergewöhnlich hohen Immunogenität des HBcAg sind rekombinante HBV CLPs attraktiv für die Verwendung als Immunogenitäts-steigernder Träger ("carrier") für Fremdantigene.

Ein alternativer Lösungsweg ist die *chemische* Kopplung von Fremdmolekülen an vorgeformte CLPs. Bei der vorliegenden Erfindung liegt eine *genetische Fusion* von Fremd-AS-Sequenzen an das Core-Protein vor. Hierbei stellte sich die Region des c/e1-Epitops noch vor der röntgenkristallographischen Strukturaufklärung anhand empirischer Versuche als bestgeeignet für die Induktion potenter B-Zellantworten heraus. Anhand der Struktur ist heute klar, dass solche Fusionen Insertionen in die Schleife ("loop") zwischen den zentralen Helices darstellen. Da dieser *loop* per se keine strukturgebende Funktion hat, kann die 3D-Struktur des Core-Proteins bei Insertion von Fremdsequenzen zumindest grundsätzlich erhalten bleiben. Abhängig von der inserierten Fremdsequenz können einige derartige Fusionsproteine weiterhin reguläre CLPs bilden, jedoch ist dies nicht immer gewährleistet, weil die Fremdaminosäuresequenz die Formung der Struktur des Fusionsproteins negativ beeinträchtigen kann. Die partikuläre Struktur ihrerseits ist essentiell für hohe Immunogenität.

Es wurden schon eine Vielzahl von Fremdsequenzen in das Core-Protein inseriert, z.T. auch die erhöhte Immunogenität experimentell nachgewiesen [Ulrich et al., Adv. Virus Res. (1998), S. 141-182]. Da viele Versuche zur Insertion von längeren Fremdsequenzen mit mehr als ca. 40 AS aber scheiterten (keine Partikelbildung mehr), nahm man an, es gebe eine natürlich begrenzte Aufnahme-Kapazität für Fremdsequenzen für bis zu 40 AS bzw. in einem speziellen Fall bis zu 120 AS.

Es wurde gefunden, daß Fremdaminosäuresequenzen, die am C- und N-Terminus mit dem Core-Protein fusioniert sind, nur dann Partikel ausbilden, wenn mindestens zwei essentielle Kriterien erfüllt sind:
(i) Die 3D-Struktur des Fremdproteins muss so geartet sein, dass sein N- und C-Terminus zur räumlichen Lage der Verknüpfungspunkte im Core-Protein (C-Terminus des N-terminalen Coreanteils, d.h. Core-AS ca. 1- bis ca. 78 [coreN]; N-Terminus des C-terminalen Coreanteils, d.h. Core-AS ca. 80 bis 149 oder 183 [coreC]) passen
(ii) falls das Fremdprotein selbst homomere Interaktionen eingeht (Dimere, Trimere, etc) muss die Struktur dieser Homo-Oligomere ebenfalls zur Struktur der beiden Core-Protein-Anteile passen

Gut geeignet für die Präsentation in vorbekannten Fusionsproteinen sind daher Fremdproteine, deren N- und C-Terminus in der nativen 3D-Struktur nahe beieinander liegen. GFP erfüllt beide dieser Voraussetzungen, viele andere Proteine aber nicht; ein Beispiel hierfür ist das outer surface protein A (OspA) des Lyme-Borreliose Erreger *Borrelia burgdorferi,* dessen N- und C-Terminus auf entgegengesetzten Seiten der langgestreckten 3D-Struktur liegen.

Die Insertion von OspA oder ähnlich ungünstig strukturierten anderen Proteinen führt zu Spannungen im Fusionsprotein. Entweder bleibt das Fremdprotein korrekt gefaltet und verhindert die Annäherung der beiden Core-Proteinanteile, so dass deren Faltung und nachfolgende Dimerisierung und Partikel bildung verhindert wird; oder die Faltung des Core-Protein-Anteils beeinträchtigt die Faltung des Fremdproteins, so dass dieses entweder nicht mehr nativ vorliegt (> veränderte Antigenität), oder - falls massiv missgefaltet - zur Aggregation führt.

Im Fall von OspA konnte dieses Problem nur partiell durch Benutzung sehr langer Verbindungssequenzen ("Linker") umgangen werden. Gerade für Vakzine-Anwendungen stellen diese Linkersequenzen ein potentielles Problem dar, da sie unerwünschte eigene Antigenität besitzen könnten, die zu schädlichen Kreuzreaktionen, bspw. mit körpereigenen Antigenen, führen können. Außerdem waren entsprechende Protein-Präparationen nur sehr begrenzt zur Bildung regulärer CLPs in der Lage.

Aufgrund der dimeren Struktur des Coreprotein-Trägers und der geometrisch begrenzten Oberfläche der Trägerpartikel könnte bei sehr großen oder stark von einer globulären Struktur abweichenden Fremdproteinen zusätzlich *generelle* sterische Hinderung ein Problem darstellen (der auf der "Kugeloberfläche" der CLPs verfügbare Raum ist begrenzt). Die Maximalgröße des Fremdproteins hängt von mehreren Faktoren ab. Mit dem erfindungsgemäßen Verfahren gelang schon die Präsentation eines ca. 320 AS umfassenden Fremdproteins (CSP).

Gegenstand der vorliegenden Erfindung ist daher ein Split-Core-Impfstoff, der als getrennte Polypeptide die Core N- und die Core C-Domäne des Core-Proteins eines Hepatitis B Virus und wenigstens eine Fremdaminosäuresequenz, gegen die eine humorale sowie gegebenenfalls zelluläre Immunantwort hervorgerufen werden soll, aufweist, wobei die Fremdaminosäuresequenz an den C-Terminus der Core N-Domäne oder an den N-Terminus der Core C-Domäne fusioniert ist und das Core-Protein capsid-like-particles binden kann. Bevorzugt werden durch das Trägersystem neutralisierende Antikörper hervorgerufen.

Wesentlich bei dem erfindungsgemäßen Split-Core-Impfstoff ist, dass das Core-Protein am c/e1 Epitop, also etwa zwischen den Aminosäuren 73 und 94 unterbrochen, also getrennt ist. Die Fremdaminosäuresequenz kann nun an das C-terminale Ende des Core N-Bereichs anfusioniert sein. In diesem Falle beginnt das N-terminale Ende des Core C-Teiles mit derjenigen Aminosäure, bei der das Core N-Protein getrennt wurde. Es ist auch möglich, dass ein Teil des c/e1 Epitops deletiert ist, dass also eine oder mehrere Aminosäuren aus dem Bereich zwischen Aminosäure 73 und 94 entfernt wurden. Alternativ hierzu ist möglich, dass die Fremdaminosäuresequenz, gegen die Antikörper gebildet werden sollen, an das N-terminale Ende des Core C-Bereichs anfusioniert ist. In diesem Falle endet der C-terminale Bereich von Core N an der Aminosäure, bei der das Core-Protein unterbrochen wurde. Ein wesentlicher Effekt, den der Split-Core-Impfstoff beibehalten muß, ist, dass die Split-Core-Impfstoffpartikel, die anfusionierte Fremdaminosäuresequenzen tragen, dennoch die corecapsid-like particles bilden können. Dies wird entweder im Saccharose-Gradienten oder in der nativen Agarose-Gelelektrophorese überprüft. Auch eine Überprüfung mit Hilfe des Elektronenmikroskops ist möglich.

Bei der Fremdaminosäuresequenz, gegen die Antikörper gebildet werden sollen, handelt es sich bevorzugt um Sequenzen, die von Oberflächenstrukturen von Mikroorganismen herstammen. Derartige Mikroorganismen rufen üblicherweise Erkrankungen beim Menschen hervor. Wenn gegen diese Strukturen neutralisierende Antikörper gebildet werden können, führt dies dazu, dass die Immunabwehr eingedrungene Mikroorganismen sehr schnell beseitigen kann. Ein Bakterium, bei dem die Impfstoffentwicklung schon relativ weit fortgeschritten ist, ist Borrelia burgdorferi, der Erreger der Lyme Disease. Bevorzugt eingesetzt werden im Rahmen der Erfindung als Fremdaminosäuresequenzen die Oberflächenproteine OspA und OspC von Borrolia burgdorferi. Bei den Fremdaminosäuresequenzen kann es sich aber auch um andere Sequenzen handeln, die von pathogenen Organismen herstammen. Ein derartiges Beispiel ist der Malaria-Erreger Plasmodium falciparum.

Bei weiteren Fremdaminosäuresequenzen, gegen die Antikörper gebildet werden können, handelt es sich um Sequenzen, die von Viren herstammen. Bevorzugt werden solche Aminosäuresequenzen eingesetzt, die von Proteinen von Viren herstammen, die mit dem Immunsystem des Wirtsorganismus in Kontakt kommen. In erster Linie handelt es sich hierbei um Oberflächenproteine, da diese in der Regel zuerst mit dem Immunsystem des Wirtsorganismus in Kontakt treten. Alternativ hierzu kann es sich aber auch um Proteine handeln, die im Verlauf des viralen Lebenszyklusses freigesetzt werden. Dabei kann es sich, je nach Virustyp, beispielsweise um Core-Proteine oder Nucleocapsidproteine eines Virus handeln.

Mit dem vorliegenden Split-Core-Impfstoff können aber nicht nur Antikörper gegen körperfremde Aminosäuresequenzen erzeugt werden, sondern auch Antikörper gegen unerwünschte körpereigene Strukturen, wie beispielsweise Tumormarker. Tumorzellen exprimieren häufig andere Oberflächen marker als gesunde Zellen. Wenn also derartige körpereigene Aminosäuresequenzen im Split-Core-Impfstoff präsentiert werden, wird die Bildung einer Immunantwort angeregt, so dass der Körper in verstärktem Ausmaß Antikörper gegen die Tumorzellen bildet. Durch diese Antikörper können die Tumorzellen leicht von der Immunabwehr erkannt und schließlich eliminiert werden. Durch das erfindungsgemäße Trägersystem kann aber auch die zelluläre Immunantwort angeregt werden, was zu einer verbesserten Wirksamkeit führen kann.

Gegenstand der vorliegenden Anmeldung ist auch ein Verfahren zur Herstellung eines Impfstoffes gegen eine heterologe Proteinsequenz. Der Split-Core-Impfstoff besteht aus zwei Teilen des Core-Antigens eines Hepatitis B-Virus, wobei entweder an die Core N-Region die heterologe Proteinsequenz anfusioniert ist, oder an das C-terminale Ende der heterologen Proteinsequenz das Core C-Protein anfusioniert ist. Diejenige Position in der Aminosäurekette, an der die beiden Domänen voneinander getrennt sind, befindet sich zwischen Position 73 und Position 94. Einzelne Aminosäuren dieser Aminosäureregion, die das c/e1 Epitop darstellen, können deletiert sein.

Das erfindungsgemäße Verfahren kann grundsätzlich auf zwei verschiedene Arten durchgeführt werden. Wenn die beiden Teile des Split-Core-Antigen als nur eine Polypeptidkette exprimiert werden, wird erfindungsgemäß eine Erkennungssequenz für eine Protease an der vorgesehenen Position eingebaut. Dadurch kann das Polypeptid nach Expression durch die Protease in zwei definierte Teile gespalten werden. Bei dieser Ausführungsform ist es auch möglich, diejenige Protease, die an der vorgesehen Schnittstelle schneidet, mitzuexprimieren. Die hierfür kodierende Gensequenz kann sich entweder auf demselben Vektor oder auf einem separaten Vektor befinden.

In einer anderen, bevorzugten Ausführungsform werden die beiden oben erwähnten Polypeptide aber mit einem speziellen Vektor exprimiert. Bevorzugt eingesetzt wird ein sogenannter bicistronischer Vektor. Dies bedeutet, dass der eine Teil des Split-Core-Impfstoffes als Polypeptidkette exprimiert wird und dass sich am C-Terminus ein Stopkodon befindet. Kurz danach können aber die Ribosomen wieder ansetzen und das zweite Polypeptid exprimieren. Dadurch wird gewährleistet, dass sich die beiden Teile des Split-Core-Impfstoffes in etwa gleicher Menge bilden und es müssen keine Vorsichtsmaßnahmen getroffen werden, damit der Wirtsorganismus nicht einen der Vektoren verliert, was dazu führen würde, dass nur noch ein Teil des Split-Core-Impfstoffes bereitgestellt wird.

Der Ausdruck "Fusionsprotein" oder "anfusioniert" bedeutet, dass zwei unterschiedliche Proteine oder Polypeptide über eine Peptidbindung miteinander verbunden sind. Solche Fusionsproteine entstehen durch Expression der dafür kodierenden, hintereinander geschalteten Nucleinsäuresequenzen.

Es wurde im Rahmen der Erfindung gefunden, daß sich als proteinchemisches Hauptproblem für eine breitere Verwendbarkeit des HBV-CLP Trägersystems (spezifisch als Impfstoff-Carrier, generell für die Präsentation von Fremdmolekülen) die beidseitige Verknüpfung des inserierten Fremdproteins über N- und C-Terminus darstellt. Läßt sich eine der beiden kovalenten Bindungen (auf der N-oder der C-terminalen Seite des Inserts) lösen, und kommt es trotzdem zur Partikelbildung, weil sich die getrennten Core-Protein-Anteile ("split-core") finden und korrekt falten, erhöht sich die Anzahl und Art der inserierbaren Fremdproteine drastisch. Dies ist ein wesentlicher Aspekt der vorliegenden Erfindung.

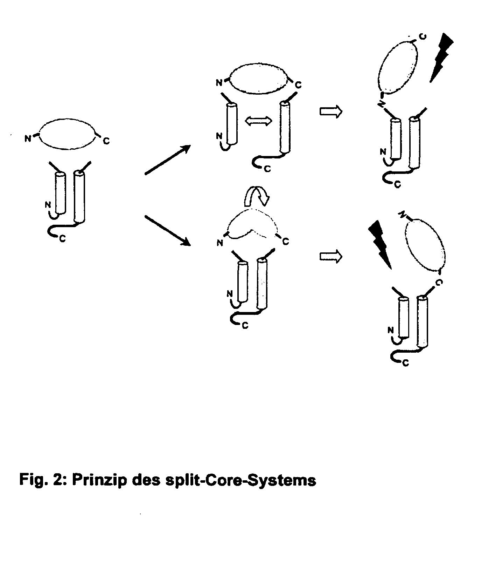

Figur 2 zeigt schematisch, dass die Insertion eines Fremdproteins mit ungünstiger 3D-Struktur (z.B. OspA) die Bildung der Partikel-bildungskompetenten Struktur des Core-Protein-Trägers verhindern kann (oberer Weg); alternativ kann die Ausbildung der korrekten 3D-Strukturs des Core-Protein-Trägers die Formation der nativen 3D-Struktur des Fremdproteins verhindern (unterer Weg). Dies würde die Bindung der gewünschten Antikörper ungünstig beeinflussen. Diese sterische Problematik wird aufgehoben, wenn eine der beiden kovalenten Verknüpfungen zwischen Fremdprotein-Insert und Träger (auf der C-terminalen oder der N-terminalen Insert-Seite) gelöst wird (Pfeil). Eine Möglichkeit der Realisierung ist die nachträgliche Spaltung eines kontinuierlichen Fusionsproteins; dies erfordert die zusätzliche Einführung einer Schnittstelle für eine spezifische Protease. Bevorzugt werden daher die beiden Fragmente von vornherein als separate Entitäten exprimiert.

Durch die erfindungsgemäße Lösung werden noch weitere Konsequenzen bewirkt:
(i) da das c/e1 Epitop auf der Partikeloberfläche liegt, entstehen auf ebendieser Oberfläche neue N-und C-Termini, die weiter derivatisiert werden können. Z.B. kann an das coreN-Fragment ein Fremdmolekül X, und an das coreC-Fragment ein Fremdmolekül Y fusioniert werden. Eine mögliche Anwendung ist die Präsentation von heterodimeren Fremdmolekülen. Hierbei muß allerdings beachtet werden, dass keine sterische Hinderung der beiden anfusionierten Teile auftreten darf.
(ii) spezifische Teilregionen einer inserierten Fremdsequenz, z.B. ein besonders wichtiges Epitop können je nach Verknüpfung über N- oder C-Terminus zur Partikeloberfläche hin, oder von ihr weg, orientiert werden. Ein Beispiel ist das sog. LA2-Epitop von B. burgdorferi OspA, das nachgewiesenermaßen im C-terminalen Bereich liegt. Antikörper, die dieses Epitop erkennen, sind neutralisierend.

Dies ist auch wichtig für die Insertion von Fremdsequenzen, die ihrerseits weitere Interaktions-Oberflächen darbieten (für die Anheftung dritter Moleküle; siehe Beispiele). Da nur einseitig verknüpft, wird der interaktionsfähigen Fremdsequenz keine artifizielle 3D-Struktur aufgezwungen; stattdessen ist sie, je nach Art, flexibel oder kann ihre korrekte 3D-Struktur ungestört einnehmen. Damit werden Interaktionen mit dem Interaktionspartner wesentlich erleichtert.

Gegenstand der vorliegenden Erfindung sind auch Arzneimittel, insbesondere Impfstoffe, die capsid-ähnliche Partikel enthalten, welche auf dem Split-Core-System basieren.

Die erfindungsgemäßen Arzneimittel beinhalten die erfindungsgemäßen Capsid-ähnlichen Partikel, wobei die Arzneimittel bevorzugt einen positiven Einfluß auf das Immunsystem aufweisen. Üblicherweise wird durch das erfindungsgemäße Split-Core-Trägersystem dem Immunsystem eine Fremdaminosäuresequenz dargeboten, gegen die das Immunsystem Antikörper, bevorzugt neutralisierende Antikörper, produziert. Diese Antikörper wirken dann maßgeblich bei der Bekämpfung des in den Körper eingedrungenen Mikroorganismus oder des Virus mit, oder sie helfen bei der spezifischen Zerstörung von unerwünschten Tumorzellen mit.

In einer weiteren Ausführungsform kann das erfindungsgemäße Split-Core-Trägersystem als diagnostisches und/oder analytisches Mittel eingesetzt werden. In den Beispielen 12 und 13 sind derartige Anwendungen näher erläutert. Bei derartigen Mitteln können beispielsweise selbstfluoreszierende, Antikörper bindende Partikel zugänglich gemacht werden, die ihrerseits Antigen binden und dieses per Fluoreszenz sichtbar machen. In einer weiteren Ausführungsform können die erfindungsgemäßen Split-Core-Trägersysteme Peptidsequenzen beinhalten, die spezifisch bestimmte Metallionen/Lantanide binden. Wenn derartige Peptide in das Split-Core-System inkorporiert werden, werden Partikel bereitgestellt, die pro einzelnem Partikel viele derartige Reporteratome/Ionen enthalten und besser nachweisbar sind.

### Beispiel 1

### Wildtyp-Core-Protein mit inserierter TEV-Protease Erkennungssequenz

In das c/e1 Epitop von wt-Coreprotein 1-149 wurde eine Erkennungssequenz für die tobacco-etchvirus (TEV) Protease eingeführt (Ersatz der AS P79+A80 durch GGGGT-ENLYFQGT-GGGG; G-Reste als Linker, um Zugänglichkeit der Erkennungssequenz für die Protease zu gewährleisten). Das rekombinant exprimierte Protein formte Partikel. Diese wurden mit TEV Protease inkubiert, der Erfolg der Spaltungsreaktion durch SDS-PAGE überprüft. Der Protease-Verdau-Ansatz wurde anschließend durch einen Sucrosegradienten sedimentiert. Es kam zur praktisch vollständigen Spaltung an der erwarteten Position, da 2 nahezu gleich große Fragmente entstanden. Trotz Spaltung sedimentierte das Protein im Gradienten etwa an dieselbe Position wie ungespaltenes wt-Core-Protein 1-149. Vergleichbare Ergebnisse wurden mit einem Fusionsprotein auf Basis des Volllängen-Core-Proteins 1-183 erhalten.

### Beispiel 2

### Core-Protein-Varianten mit größeren inserierten Fremdsequenzen

Als Beispiel für ein Fusionsprotein mit moderat großer Insertion wurde in das c/e1-Epitop die Sequenz für ein artifizielles Peptid "ACID" (flankiert von Gly-reichen Linkern; gesamt 65 AS) inseriert, welches mit einem komplementären Peptid "BASE" interagieren kann [O'Shea et al., Curr.Biol. (1993), 658-667]; daran anschließend die TEV-protease-Erkennungssequenz GGGGSGGGVEDGGGGSGGGGT-*AQLEKELQALEKENAQLEWELQALEKELAQTG-*ENLYFQGTGGGG.

Das Fusionsprotein bildete CLPs. Diese wurden isoliert und wie oben mit TEV-Protease inkubiert. Auch hier kam es zu spezifischer Spaltung, die Partikel blieben aber intakt. In diesem Fall sind die TEV-Spaltfragmente unterschiedlich groß und daher im SDS-Gel direkt zu unterscheiden. Trotz Spaltung ko-sedimentieren die Fragmente in die CLP-spezifischen Gradienten-Fraktionen.

Dieses Beispiel zeigt, dass sich auch CLPs aus Core-Protein mit einem inserierten 65 AS Peptid (Linker + ACID + TEV-Schnittstelle) spezifisch spalten lassen, die partikuläre Struktur bleibt trotzdem erhalten.

### Beispiel 3

Als weiteres Beispiel ist ein entsprechender Versuch mit einem Core-Protein gezeigt, das die AS Sequenz 18-273 des Borrelia burgdorferi OspA Proteins enthält. Das entsprechende Protein ohne TEV-Spaltstelle ist in Nassal et al., 2005 beschrieben; nur ein Bruchteil davon bildete reguläre Partikel, was sich auch in einer breiten Verteilung im Sucrosegradienten gezeigt hatte. Der nicht geschnittene Impfstoff ist also als solcher nur bedingt zu gebrauchen. Da zudem sehr lange Extra-Linkersequenzen mit eigenem, unerwünschten Antigenpotential notwendig waren, um wenigstens einen kleinen Anteil der Präparation in CLP-Form zu erhalten, wäre dieser nicht geschnittene Impfstoff nicht im Menschen/medizinisch anwendbar.

Nach Einführung einer TEV-Spaltstelle wurde das Fusionsprotein wie in Beispiel 2 mit TEV-Protease behandelt, dann im Sucrosegradienten sedimentiert. Auch hier kam es zur spezifischen Spaltung, allerdings nicht vollständig (vermutlich durch sterische Hinderung via OspA schlechtere Zugänglichkeit der TEV-Schnittstelle); Zuordnung der Fragmente mittels monoklonaler Antikörper, die spezifisch den N-terminalen bzw. C-terminalen Coreanteil erkennen; der a-coreN Antikörper reagiert demzufolge mit dem ungespaltenen Fusionsprotein sowie mit dem N-terminalen Spaltfragment. Trotzdem zeigte das partiell gespaltene Material ein Partikel-typisches Laufverhalten.Diese Daten deuteten darauf hin, dass durch Öffnen einer der beiden Verbindungen zwischen inseriertem Fremdprotein und Coreträger die Partikelbildung deutlich verbessert werden konnte.

Das Beispiel belegt, dass sich auch ein Corefusionsprotein mit einer *großen* Fremdprotein-Insertion (255 AS OspA + Linker + TEV-Schnittstelle) spezifisch spalten läßt (wenn auch weniger effizient). Während das zuvor beschriebene kontinuierliche coreOspA Fusionsprotein sich breit über den Gradienten verteilt (Nassal et al., 2005), reichert sich das (partiell) gespaltene Fusionsprotein distinkt in partikeltypischen Gradientenfraktionen an. Dies ist ein Indiz für verbesserte Partikelbildungseffizienz durch die Spaltung.

In einem weiteren Versuch konnte gezeigt werden, dass (von einem kompatiblen Plasmid) ko-exprimierte TEV-Protease zur Spaltung von TEV-Schnittstellen tragenden Corefusions-Proteinen führt, teilweise effizienter als durch nachträgliche in vitro Spaltung möglich. Auch für die schon in den Bakterien gespaltenen Fusionsproteine konnte Partikelbildung via Sucrosegradienten-Sedimentation nachgewiesen werden.

Die Beispiele 1 bis 3 zeigten, dass *vorgeformte* CLPs nach Spaltung im Bereich des c/e1 Epitops intakt bleiben. Dies ist strukturell damit zu erklären, dass die Verbindungsschleife zwischen den spike-bildenden Helices keine per se strukturgebende Rolle hat, konnte so aber nicht erwartet werden. Dies führte zu der bevorzugteren Ausführungsform, bei der, statt nachträglicher Spaltung der kontinuierlichen Proteinkette die coreN und coreC-Teile direkt als separate Proteinfragmente exprimiert werden, wobei sie sich offensichtlich spontan zusammenfinden und Partikel bilden. Ein solcher Ansatz hat mindestens drei wesentliche Vorteile:
(i) Vereinfachung: der zusätzliche Spaltungsschritt mit TEV-(oder einer anderen) Protease ist nicht mehr notwendig
(ii) keine zusätzliche Peptidsequenz im Fusionsprotein für die spezifische Protease-Erkennung notwendig
(iii) eine - wenn auch nur partielle - Partikelbildung mit sterisch ungünstigem Fremdprotein wie OspA erforderte sehr lange Linkersequenzen; diese sind verzichtbar, wenn sterische Hinderungen durch die nicht mehr beidseitig notwendige Verknüpfung mit dem Core-Trägerprotein vermieden würden.

Die Punkte (ii) und (iii) sind besonders für Vakzineanwendungen bedeutsam, da jegliche zusätzliche Sequenz zu unvorhersehbaren immunologischen Konsequenzen führen kann (neue Epitope durch die Zusatzsequenzen, möglicherweise Kreuzreaktionen mit körpereigenen Epitopen)

### Beispiel 4

### Expressionkonstrukte zur annähernd äquimolaren Expression von coreN und coreC Fragmenten

In der kontinuierlichen Peptidkette liegen die späteren Spaltprodukte zwangsläufig in äquimolaren Mengen vor. Bei separater Expression sollten die beiden Teile daher für eine effiziente Zusammenlagerung ebenfalls in annähernd äquimolaren Mengen entstehen. In ersten Versuchen wurden zwei separate, kompatible Plasmide zur Expression der beiden Trägerprotein-Teile eingesetzt, allerdings mit mäßigem Erfolg.

In bevorzugter Ausführungsform werden bicistronische Vektoren eingesetzt, die zudem den Vorteil bieten, dass auf Selektion mit einem weiteren Antibiotikum (für den Erhalt des ansonsten notwendigen zweiten Plasmids) verzichtet werden kann.

**Typ1 Konstrukte** enthalten hinter einem gemeinsamen Promotor (hier T7 Phagen RNA Polymerase Promotor) zwei Expressionskassetten mit jeweils einer vorgeschalteten Ribosomen-Bindungsstelle (ribosome binding site, RBS; "Shine-Dalgarno-Sequenz"). In den hier realiserten Vektoren wird die Translation des coreN Fragments durch ein artifizielles Stop-Codon nach AS Prolin 79 (P79) beendet, die Translationsinitiation des 2. Fragmentes durch ein artifizielles Start-Codon vor AS Serin81 (S81) ermöglicht. Eine solche Operonstruktur ist in Bakterien häufig zu finden ("polycistronische mRNAs"; Ribosomen können relativ unabhängig an jede RBS auf der mRNA binden und die Translation des 3' gelegenen Gens initiieren). In den realisierten Konstrukten ist die zweite RBS eine exakte Kopie der ersten RBS, die aus dem originalen pET-Vektoren stammt; andere RBS-Sequenzen sind mit Sicherheit ebenfalls möglich. Das ATG Startcodon des 2. Cistrons ist Teil einer Nde-Schnittstelle (CATATG) für das leichte Einklonieren von Fremdsequenzen.

**Typ2 Konstrukte** ("stop/start") enthalten keine separate zweite RBS; stattdessen überlappt mit dem Stopcodon des *upstream* Gens (NNN TGA) ein Inititionscodon für das *downstream* Gen (NNA TGA) in einem um 1 verschobenen Leseraster. In diesem Fall können Ribosomen nach Ablesen des ersten Gens direkt am ATG des zweiten Gens re-initiieren; eine solche Anordnung erlaubt die Koexpression zweier Gene; ahnliche Stop-Start Arrangements finden sich in einigen Bakteriophagen. Sie ist auch in einigen eukaryonten non-LTR Retrotransposons verwirklicht (Verbindung ORF1/ORF2). Anhand der bekannten Sequenzen z.B. im E. coli Trp Operon sollten andere Kombinationen von nahe benachbarten, oder überlappenden Stop-/Startcodons (z.B. TAA TG, TGA TG, etc.) ähnlich geeignet sein.

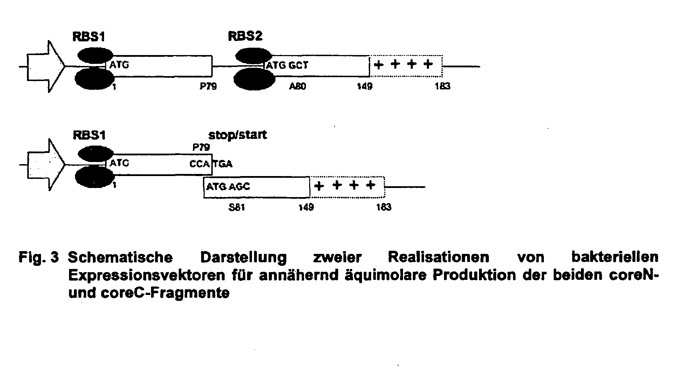

Bevorzugte Expressionsvektoren sind schematisch in Fig. 3 dargestellt.

Oben: Typ 1 Konstrukte. Ein Promotor, z.B. für T7 RNA Polymerase, führt zur Transkription einer bi-cistronischen mRNA (für effiziente Termination kann 3'-seitig eine Terminatorsequenz eingefügt sein). Das erste Cistron kodiert die HBV Coreprotein-AS-Sequenz 1-79. Eine vorgeschaltete Ribosomen-bindungsstelle (RBS1) ermöglicht die Translationsinitiation (kleine und große Ribosomen-Untereinheit als Ovale dargestellt). Am 3'-Ende des Codons für P79 folgt ein Stop-Codon. Am 5'-Ende des zweiten Cistrons liegt eine weitere RBS (RBS2) für die Translationsinitiation des coreC-Fragments. Dieses verfügt dazu über ein eigenes Initiationscodon (ATG). Im gezeigten Beispiel beginnt die coreC-Sequenz mit Ala80; sie kann sich bis AS-Position 140, besser 149, oder bis zum authentischen Ende bei Pos. 183 erstrecken. Die AS-Sequenz ab Pos. 140, besser 149, kann auch durch Fremdsequenzen ersetzt werden.

Unten: Typ2 Konstrukte. Diese enthalten nur eine RBS vor dem ersten Cistron. Die Translation des coreC-Fragments erfolgt durch Re-Initiation, indem das artifizielle Startcodon von coreC (ATG) mit dem Stop-Codon von coreN (TGA) wie gezeigt überlappt. Im Bespiel beginnt die coreC-Sequenz mit Ser81.

Die AS 77, 78 und 79 des Core-Proteins sind Glutamat (E; Codons: GAA, GAG), Aspartat (D; Codons: GAC oder GAT), AS 79 Prolin (P; Codons: CCN). In den realisierten Typ 1 Konstrukten wurde für die AS-Sequenz EDP die Nukleotid-Sequenz GAG GAT CCN gewählt, wodurch eine Schnittstelle für BamHI entsteht (GGATCC); durch diesen singulären BamHI Schnitt ist eine simple Einklonierung von unterschiedlichen coreN- und coreCkodierenden DNA-Fragementen möglich. Das Nucleotid N im Prolin Codon CCN ist nicht festgelegt; das Stop-Codon kann TAA, TAG, oder TGA sein.

In Typ 2 Konstrukten lautet die Sequenz bis zum Ende des ersten Gens G GAT CCa TGA. Auch hier ist eine BamHI Schnittstelle (unterstrichen) für das Einklonieren unterschiedlicher coreN bzw. coreC kodierender DNA-Fragmente vorhanden. Für P79 muss in den realisierten Typ2-Konstrukten das Codon CCa sein, das Stop-Codon muss TGA (> CCa TGA) sein, um das Entstehen eines überlappenden ATG Initiationscodons für das zweite Gen zu erlauben. Das Codon für die erste AS des zweiten Gens nach dem ATG-Startcodon muss mit A beginnen. Alternative Stop/Start Nt-Folgen wären TGA TG oder TAA TG wie im E. coli Trp Operon zwischen TrpE und TrpD oder auch TrpB und TrpA [Das and Yanofsky (1989), Nucl. Acids Res., S. 9333-9340; Oppenheim and Yanofsky (1980), Genetics, S. 785-795].

In den realisierten Vektoren (pET28a2-xxx) beruht das Grundgerüst auf dem kommerziellen Vektor pET-28, bei dem jedoch das Kanamycin-Resistenzgen gegen dasjenige für Ampicillin-Resistenz ausgetauscht wurde. Mit Sicherheit sind jedoch andere Vektor-Grundgerüste ebenfalls anwendbar (verschiedene replication oris, verschiedene Resistenzgene, verschiedene Promotoren, verschiedene RBS). Essentiell ist die bicistronische Anordnung der Gene für coreN und coreC, entweder durch 2 separate RBS, oder durch Stop/Start Anordnung. In eigenen Versuchen liessen sich mehrere split-Core Proteine von Typ1 wie von Typ 2-Konstrukten exprimieren. Für Typ 2 (Stop/Start) Konstrukte war dies jedoch in einer geringeren Anzahl von Konstrukten der Fall. Ausserdem ist die mögliche Sequenzvariabilität bei Typ2-Konstrukten geringer (s.o.). Typ1 Konstrukte sind daher breiter anwendbar.

Als C-terminale AS des coreN-Anteils wurde P79 gewählt, da Prolin besonders Proteaseresistent ist und somit eine Degradation des Fragments minimiert wird. Aufgrund der bekannten 3D-Struktur sowie beschriebener Deletionsvarianten in dieser zentralen Region lässt sich aber annehmen, dass
(i) das Ende des coreN Fragments im Bereich der AS ca. ab Pos. 72 - 74, besonders Gly73
(ii) der Beginn des coreC Fragments im Bereich der AS ca. ab Pos. 78-86, möglicherweise bis Gly94 liegen könnte, ohne dass wesentlich andere Ergebnisse als mit den hier gewählten Grenzen (coreN Ende bei P79, coreC Start bei S81, mit vorgeschaltetem Start-ATG) erhalten würden.

Für die Expression in eukaryonten Zellen sind die beschriebenen Konstrukte aufgrund des unterschiedlichen Transkriptions- und Translations-Mechanismus so nicht anwendbar; statt des prokaryonten Promotors muß ein eukaryonter Promoter verwendet werden (z.B. CMV-IE und viele andere), sowie ein Poly-Adenylierungssignal (z.B. aus SV40 Virus, oder viele andere) nach dem Ende des/der offenenen Leseraster(s) eingeführt werden.

Eine annähernd gleiche Expression von coreN und coreC in Eukaryonten kann erreicht werden durch:
(i) Ko-Transfektion zweier Plasmide (je eines für coreN, eines für coreC);
(ii) Transfektion eines Plasmids mit 2 unabhängigen Expressions-Kassetten;
(iii) Transfektion eines Plasmids mit funktionell bicistronischer mRNA; z.B. könnte das zweite Gen durch eine vorgeschaltete "internal ribosome entry site" (IRES) exprimiert werden;
(iv) Möglicherweise ist auch ein Typ2-Vektor-artige Stop-/Start Anordnung in Eukaryonten funktionell.

### Beispiel 5

### Separate Expression von wt-coreN und coreC Fragmenten führt zur Bildung intakter CLPs

Mittels Expressionsvektoren auf Typ1-Basis (Fig. 3) wurde Wildtyp-Core-Protein in zwei Teilen in E. coli BL21 Zellen exprimiert, einmal auf Basis der AS 1-149, einmal AS 1-183. Beide erwarteten Fragmente wurden gebildet und lagerten sich zu intakten CLPs zusammen. Dies wurde mit Sucrose-Gradienten sowie nativ-Agarosegel-Elektrophorese nachgewiesen. Als direkter Beweis für die Bildung von CLPs wurde ein elektronenmikroskopischer Vergleich (negative staining) von Partikeln aus kontinuierlicher AS 1-183 Kette mit C-terminalem His6-tag vs. Partikeln aus dem entsprechenden split-Core-System durchgeführt. Bei dieser Auflösung waren keine Unterschiede detektierbar.

Wt-HBV-CLPs sind sehr robust und beständig gegen Harnstoff in molaren Konzentrationen; CLPs aus C-terminal trunkiertem Core-Protein 1-149 lassen sich durch 3-5 M Harnstoff in Dimere dissoziieren und durch Änderung der Pufferbedingungen (Entfernen des Harnstoffs, neutraler pH, erhöhte Salzkonzentration) in vitro wieder zu CLPs reassoziieren. Eine vergleichende Untersuchung zur Stabilität der CLPs aus split-Core zeigte eine ähnlich hohe Stabilität der split-Core CLPs. Dies zeigt die hohe Affinität der coreN und coreC-Anteile zueinander, und ist wichtig für die Verwendung als Trägersystem für Fremdmoleküle.

### Beispiel 6

### Separate Expression von Fremdprotein-fusionierten-coreN und wt-coreC Fragmenten, sowie vice versa, führt zur Bildung intakter CLPs

Zum Nachweis der grundsätzlichen Eignung des split-core-Systems für die CLP-Bildung und Präsentation von Fremdproteinen wurde als Modell GFP entweder an coreN oder an coreC fusioniert; für das coreN-Konstrukt wurde entweder ein Typ1 (zweite RBS) oder Typ2 (Stop/Start) Vektor benutzt. Alle Konstrukte führten zur Expression grün fluoreszierender Proteine, die laut Sedimentation in Sucrosegradienten Partikel bildeten.

Zum weiteren Nachweis der physikalischen Assoziation der jeweiligen coreN und coreC Fragmente wurden Aliquots der Gradientenfraktionen der Elektrophorese in nativen Agarosegelen unterzogen. Dabei bleiben Partikel erhalten, ebenso der GFP-Chromophor. In den Fraktionen fand sich sowohl bei GFP an coreN, wie GFP an coreC, eine distinkte, grün fluoreszierende Bande; nicht assembliertes GFP Fusionsprotein verblieb in den oberen Gradientenfraktionen, und zeigte ein anderes Laufverhalten sowie eine diffusere Verteilung (schnellere Diffusion im Gel aufgrund der wesentlich kleineren Struktur im Vergleich zum Partikel aus 180 oder 240 Untereinheiten). Sowohl für GFP an coreN wie an coreC ließ sich mit dem a-coreC Antikörper in der grün fluoreszierenden (dito GFP enthaltenden) Bande auch coreC nachweisen.

Dieses Beispiel zeigt zweierlei, nämlich
(i) Im split-core-System kann ein Fremdprotein von ca. 240 AS, hier GFP, sowohl an coreN wie an coreC fusioniert werden, ohne die CLP-Bildung zu stören.
(ii) Sowohl Typ1 wie Typ2 split-Core-Vektoren sind für die Ko-Expression von coreN und coreC mit anfusionierter Fremdsequenz geeignet.

### Beispiel 7

### CLP-Präsentation von medizinisch relevanten Fremdproteinen im splitcore-System, die kowentionell nicht, oder nur sehr eingeschränkt, möglich ist

### Borrelia burgdorferi OspA

Wie eingangs dargelegt, führt die im bisherigen System erforderliche beidseitige Verknüpfung des inserierten Fremdproteins mit dem Trägerprotein zu starken topologischen Einschränkungen. Während GFP eine natürliche "Passform" hat, trifft dies für OspA nicht zu. Als Präzedenz-Fall für ein ungünstig strukturiertes Fremdprotein wurde daher OspA im split-Core-System eingesetzt, und entweder an coreN oder coreC fusioniert. Im Gegensatz zum früheren kontinuierlichen Konstrukt [beschrieben in Nassal et al., 2005] mit breiter Verteilung im Gradienten reicherten sich beide Fusionsproteine distinkt in den Partikel-typischen Fraktionen an. Eine Elektronenmikroskopie-Analyse zeigte eine drastisch verbesserte CLP-Bildungseffizienz verglichen zum alten, kontinuierlichen Konstrukt, sowohl bei Fusion von OspA an coreN wie an coreC.

Dieses Beispiel belegt, daß
(i) das split-core-System die CLP-Präsentation von Fremdproteinen erlaubt, deren Struktur im herkömmlichen kontinuierlichen Core-System mit der CLP-Bildung interferiert,
(ii) im split-Core-System effiziente CLP-Bildung durch Fusion des Fremdproteins sowohl an coreN wie auch coreC möglich ist.

In analoger Weise ließ sich auch Borrelia burgdorferi OspC erfolgreich im erfindungsgemäßen Split-Core-System präsentieren.

### Beispiel 8

### Circumsporozoiten-Protein des Malaria-Erregers Plasmodium falciparum

Ein weiteres unter Vakzine-Gesichtspunkten hochrelevantes Pathogenprotein ist das Circumsporozoiten-Protein (CSP) des Malaria-Erregers *Plasmodium falciparum.* CSP (hier verwendete Form: 319 AS Gesamtlänge) enthält eine ca. 110 AS lange Wiederholungssequenz der Tetrapeptid-Motive NANP/NVDP. Die Struktur von CSP ist nicht bekannt; die C-terminale ca. 50 AS Domäne mit 4 Cys-Resten hat wahrscheinlich (aufgrund Sequenz-Homologie) eine ähnliche Faltung wie Thrombospondin Typ1 repeats. Die repeat-Sequenz ist immunogen, möglicherweise enthalten aber auch die anderen Regionen von CSP wichtige Epitope. Im konventionellen kontinuierlichen Coresystem konnte das Volllängen-CSP nicht in CLP-Form präpariert werden. Dagegen war dies sehr wohl mit dem split-Core-System möglich. Eindeutige Daten liegen derzeit für die Fusion von CSP an coreN vor; bei Koexpression mit coreC (sowohl als 149 wie als 183-Konstrukt) bilden sich effizient partikuläre Strukturen.

Es wurde eine Serie von Konstrukten (sowohl auf Basis von core 1-149 wie 1-183) hergestellt, die entweder nur die repeat-Sequenz, oder trunkiertes CSP ohne Cys-reiche Domäne jeweils im kontinuierlichen oder split-Core-System enthalten; dazu komplettes CSP fusioniert an coreN. Die CLP-Präparationen wurden in einer vergleichenden Immunogenitäts-Studie in Mäusen eingesetzt, die aber noch nicht abgeschlossen werden konnte. Die bisherigen Daten zeigen, dass der im Split-Core-System hergestellte Impfstoff überlegene Eigenschaften aufweist. Dies ist darauf zurückzuführen, dass das komplette CSP ausschließlich bei Anwendung des erfindungsgemäßen Trägersystems die Fähigkeit zur CLP-Bildung aufweist. Bei anderen Systemen entstehen dagegen keine CLPs. Daher dürften mit Hilfe des erfindungsgemäßen Trägersystems hergestellte CSP-Impfstoffe eine überlegene Immunogenität aufweisen und insbesondere neutralisierende Antikörper induzieren.

### Beispiel 9

### Split-Core-CLPs als Träger für Fremdproteine verstärken die B-Zell-Antwort

### gegen das Fremdprotein

In einer Immunogenitätsstudie in Mäusen werden 5 core-OspA-Konstrukte aus dem split-Core-System vergleichend untersucht mit nicht an Core fusioniertem, lipidiertem OspA (LipOspA); dieses ist die Basis der kommerziellen Lymerix-Vakzine, daher der derzeitige "Goldstandard" für einen Lyme-Krankheits-Impfstoff. Der Lipidanteil in LipOspA (Tris-Palmitoyl-Cystein-(Pam3-Cys)) ist essentiell für dessen relativ hohe Immunogenität, nicht lipidiertes OspA ist nur sehr schwach immunogen (vgl. Nassal et al, 2005). Drei der core-OspA-Konstrukte enthalten Volllängen-OspA (AS 18-273), zwei weitere verkürztes OspA (beginnend bei AS 185, bis AS 273).

6 Gruppen von je 5 BALB/c Mäusen wurden dazu mit je 10 µg Antigen 4-mal immunisiert (Tag 0, 14, 29, 49):
- Gruppe 1:: LipOspA
- Gruppe 2:: Volllängen-OspA (AS 18-273) an coreN, coreC von Core-AS 81 bis 183
- Gruppe 3:: Volllängen-OspA (AS 18-273) an coreN, coreC von Core-AS 81 bis 149
- Gruppe 4:: Verkürztes OspA (AS 185-273) an coreN, coreC von Core-AS 81 bis 183
- Gruppe 5:: Verkürztes OspA (AS 185-273) an coreN, coreC von Core-AS 81 bis 149
- Gruppe 6:: Volllängen-OspA (AS 18-273) an coreC bis Core-AS 183, coreN von AS 1-79

Zur Bestimmung der induzierten Antikörper wurde Blut entnommen an Tag -1 (= 1 Tag vor der ersten Immunisierung; = Präimmunserum), sowie an den Tagen 8, 26, 36 und 57. Mittels ELISA wurden bestimmt: die Kinetik der Bildung OspA-spezifischer Antikörper; der jeweilige Anteil LA2-äquivalenter Antikörper, wobei LA2 ein monoklonaler, bekannt neutralisierender Antikörper ist, der ein komplexes konformationelles Epitop aus diskontinuierlichen AS-Sequenzen *downstream* von AS 185 von OspA erkennt.

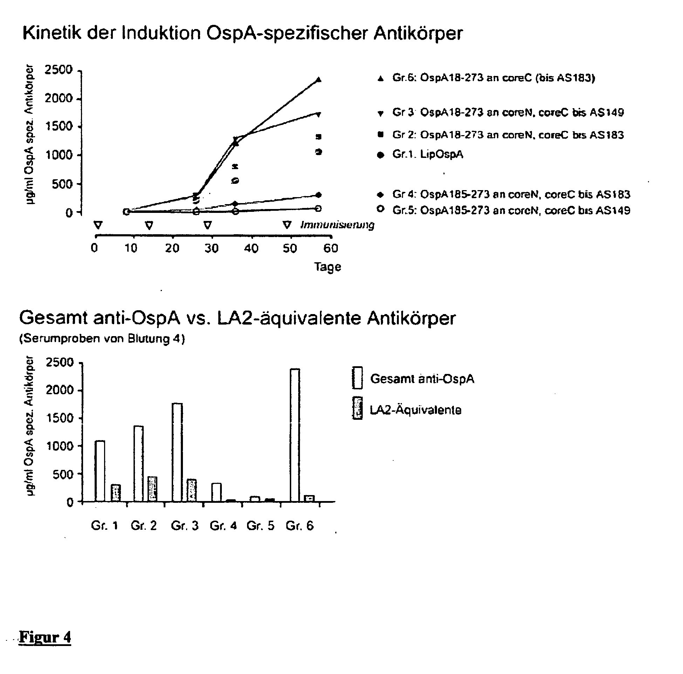

Die Ergebnisse dieses Versuches sind in Fig. 4 detailliert dargestellt.

Fig. 4A zeigt die Kinetik der Induktion von Gesamt-anti-OspA Antikörper (in µg spezifischen Ak pro ml Serum). Bereits nach der zweiten Immunisierung enthalten die Seren aus Mäusen immunisiert mit den Volllängen OspA split-Core Konstrukten nachweisbare und mit den durch LipOspA hervorgerufenen vergleichbare anti-OspA Titer. Nach der 3. und 4. Immunisierung übersteigen die anti-OspA Titer in den split-Core Volllängen-OspA immunisierten Mäusen deutlich diejenigen der mit LipOspA immunisierten Mäuse; besonders hoch sind die Titer nach Immunisierung mit OspA18-273 an coreC (Gruppe 6). Verkürztes OspA185-273 ruft im Kontext von core149 (Gruppe 5) und core183 (Gruppe 4) nur eine wesentlich geringere OspA-spezifische Antwort hervor.

Fig. 4B vergleicht direkt die Gesamt-anti-OspA Ak-Titer mit denjenigen, die gegen das bekannt neutralisierende LA2-Epitop im C-terminalen Bereich von OspA gerichtet sind. Volllängen-OspA an coreN, sowohl im Kontext von core149 wie core183 (Gruppe 2 bzw. 3) induzieren höhere anti-LA2-äquivalente Ak-Titer als LipOspA, Volllängen-OspA an coreC deutlich niedrigere. Eine Untersuchung zum jeweiligen Gehalt an *neutralisierenden* Ak (Protektion gegen challenge mit dem *B.burgdorferi* Erreger) wird weiterhin durchgeführt.

Das Beispiel zeigt, dass split-Core CLPs spezifische Antikörper gegen das präsentierte Volllängen-OspA Fremdprotein induzieren, wobei die Titer höher sind als mit dem etablierten lipidierten LipOspA, bei dem der Lipidanteil wesentlich für die Immunogenität ist. Der unterschiedliche Anteil LA2-äquivalenter Ak nach Immunisierung mit OspA an coreN (ca. 30 %) vs. OspA an coreC (< 5 %) zeigt, dass die Art der Verknüpfung die Art der entstehenden Ak beeinflusst; siehe dazu Beispiel 10.

### Beispiel 10

### Gezielte Induktion regionsspezifischer Antikörper gegen ein Fremdprotein durch alternative Fusion entweder an coreN oder an coreC

Bei Proteinen, deren N- und C-Terminus nicht unmittelbar benachbart sind wie in GFP, führt die Fusion an coreN zu einer anderen Orientierung relativ zur Partikeloberfläche als die Fusion an coreC (coreN: C-Terminus des Fremdprotein "out"; coreC: N-Terminus des Fremdproteins "out"). Es ist zu erwarten, dass bei Verwendung als B-Zell-Vakzine die jeweils am weitesten ins Lösungsmittel ragenden Proteinanteile die stärkste Antikörperantwort hervorrufen. Somit lassen sich unterschiedliche regionsspezifische Antikörper induzieren, indem das Fremdprotein entweder an coreN (C-terminale Seite des Fremdproteins "out") oder an coreC fusioniert wird (N-terminale Seite des Fremdproteins "out").

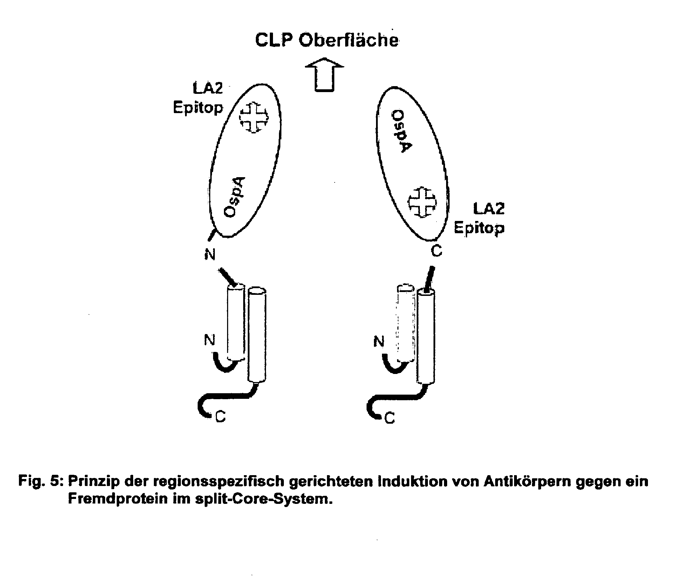

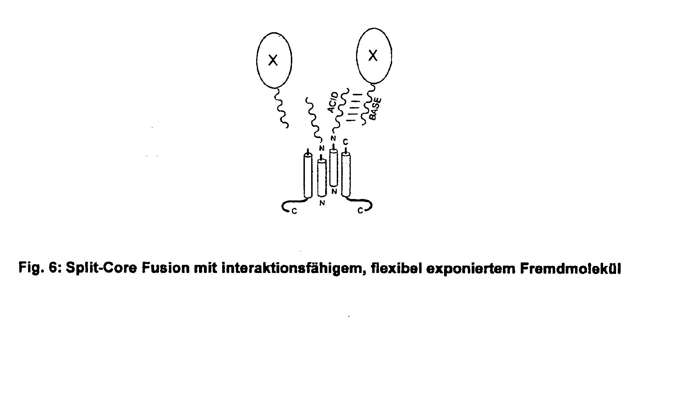

Figur 5 zeigt schematisch, wie bei einem Fremdprotein mit gestreckter Struktur, wie hier OspA, über die Fusion an entweder coreN, oder coreC, bestimmt werden kann, welcher Teil des Moleküls am meisten von der CLP-Oberfläche weg zeigt. Ein gut kartiertes Epitop in OspA ist LA2 im C-terminalen Bereich, das von einem neutralisierenden monoklonalen Antikörper, LA2, erkannt wird. Wie erwartet, induzieren split-Core-OspA CLPs mit OspA an coreN (daher OspA C-Terminus exponiert) eine starke LA2-äquivalente Antwort, CLPs mit OspA an coreC nur eine schwache LA2-äquivalente Antwort. Dafür ist die Antwort gegen andere Bereiche von OspA verstärkt.

Für OspA als Fremdprotein war zu erwarten, dass eine Fusion an coreN das LA2-Epitop im C-terminalen OspA Bereich gut zugänglich macht, eine Fusion an coreC dagegen die bislang immunologisch nicht gut charakterisierte N-terminale OspA Region. Die oben gezeigten Daten bestätigen eindrücklich dieses Konzept (mit OspA an coreC sehr hohe anti-Gesamt-OspA Titer, aber nur geringe LA2-äquivalente Ak-Titer).

Das split-core-System besitzt also eine hohe immunverstärkende Aktivität, sowohl bei Fusion des Fremdproteins an coreN wie an coreC.

Durch Wahl des Verknüpfungsortes (coreN vs. coreC) kann die Art/Regionsspezifität der induzierten Antikörper gesteuert werden. Für optimale Impfergebnisse können Mischungen von Split-Core-Impfstoffen eingesetzt werden, bei denen die heterologe Fremdaminosäuresequenz einmal am Core N und einmal am Core C Teil anfusioniert sind, so dass verschiedene Epitope optimal präsentiert werden.

### Beispiel 11

### Fusionen mit Bedeutung jenseits von Vakzine-Anwendungen

Neben der direkten Bedeutung als immunverstärkender partikulärer Träger für Peptid- und hier besonders Protein-Antigen-Vakzine sind eine Vielzahl weiterer Anwendungen für eine solche Träger-Plattform vorstellbar. Immer wird eine große Anzahl (180 oder 240) präsentierter Moleküle in eine nahe, symmetrische Nachbarschaft gebracht: Handelt es sich um interaktionsfähige Fremdmoleküle (Vakzine stellt einen Spezialfall dar: Interaktion mit Antikörpern), so hat der Partikel mit vielen Kopien des Fremdmoleküls eine drastisch erhöhte Avidität verglichen zum monomeren Fremdmolekül (vgl. natürliche IgM Pentamere; auch MHC-Tetramere in der Immundiagnostik). Damit solche interaktionsfähigen Fremdmoleküle mit ihren Interaktionspartnern reagieren können, müssen sie auf der CLP-Oberfläche zugänglich sein. Dies ist im split-Core-System wesentlich erleichtert gegenüber dem konventionellen kontinuierlichen System.

Eine Reihe von Modell-Fremdsequenzen wurde im split-Core-System exprimiert, alle getesteten Insertionen bildeten CLPs. Für einige wurde die Interaktionsfähigkeit direkt nachgewiesen.

Das split-Arrangement macht auf der CLP-Oberfläche neue Termini für weitere Derivatisierung zugänglich. Die Figur 5 zeigt ein split-Core Dimer mit an coreN fusioniertem ACID-Peptid, welches mit dem komplementären BASE-Peptid interagieren kann. Wurde das BASE-Peptid an ein Molekül X fusioniert, bindet X an die ACID-CLPs. Durch die split-Anordnung ist ACID flexibel; ACID inseriert im kontinuierlichen Core-System interagiert mit BASE-Peptid, da sich aber eine starre coiled-coil Doppelhelix zwischen ACID- und BASE-Peptid ausbildet [O'Shea et al., 1993], wird die Struktur des Core-Trägers instabil, die CLPs zerfallen. In der split-Anordnung sollten die CLPs dagegen stabil bleiben. Diese Koppelung ist generalisierbar: Statt des ACID-Peptides kann ein anderes interaktionsfähiges Molekül A an coreN, oder coreC fusioniert sein. Entsprechende CLPs können dann mit B, einem spezifischen Interaktionspartner von A, interagieren. Solche Interaktionspartner-Paare sind His6 - Ni-NTA, Biotin-Akzeptor-Peptid - Streptavidin; Z33 - Immunglobulin.

Andere Koppelungsmöglichkeiten sind nachfolgend näher erläutert:

### a) His6-tag:

An coreN via Gly2-Linker fusioniert; bildet Partikel, die an Ni2⁺NTA Agarose binden. His-tag im Gegensatz zum kontinuierlichen System frei zugänglich. Vergleichbares Insert (His7) in c/e1 des kontinuierlichen Core-Systems ergab keine nennenswerten Mengen Protein, weil dort der His6-tag Rest sterisch nicht zugänglich ist.

### b) Biotin-Akzeptor (BA)-Peptid:

Biotin-Akzeptor (BA23) ist ein artifizielles 13 AS langes Peptid (GLNDIFEAQKIEWH)), welches durch die Biotin-Ligase BirA aus E. coli biotinyliert wird. Effiziente Biotinylierung erfordert freie Zugänglichkeit; diese ist im split-core-System gegeben, im kontinuierlichen core-System nicht oder nur eingeschränkt. Das split-Core-BA Fusionprotein bildet CLPs und wird in E. coli biotinyliert. An das CLP-präsentierte Biotin kann Avidin/Streptavidin bzw. deren Konjugate gebunden werden.

### c) Z 33 Domäne aus Protein A

S. aureus Protein A bindet Immunglobuline mit hoher Affinität ("Protein-A-Sepharose" für Immunpräzipitation). Protein A besteht aus 5 strukturell ähnlichen Ig-Bindedomänen. Z33 ist eine einzige solche Domäne mit modifizierter AS-Sequenz; Gesamtlänge 33 AS). Z33 hat immer noch hohe Affinität zu Ig's (Kd 40 nM). Die Bindung an unterschiedliche Ig's setzt strukturelle Flexibilität voraus, zudem Zugänglichkeit. Die Z33 Sequenz wurde über eine lange Linkersequenz an coreN fusioniert:
*GGGGSGGGVEDGGGGSGGGGT*-FNMQQQRRFYEALHDPNLNEEQRNAKIKSIREDP.

Im split-core-System bildeten sich CLPs. Nach Zugabe eines FITC-markierten Immunglobulins und erneuter Sedimentation konnte ein Anteil der Fluoreszenz in den Partikel-typischen Gradientenfraktionen nachgewiesen werden. Dies war nicht der Fall, wenn statt der Z33-Fusion wt-Core-Protein CLPs eingesetzt wurden.

### d) ACID Peptid Insert

Das ACID-Peptid inseriert in das kontinuierliche core-System interagiert mit dem komplementären BASE Peptid ("Peptid-Velcro"= Peptid-Klettverschluss) via coiled-coil Formation der Peptide; [O'Shea et al., 1993] unter massiven Veränderungen der Corestruktur bis hin zum Zerfall; Ursache ist, dass das ACID Peptid alleine eine flexible Struktur annimmt, die keinen "Stress" auf die Corestruktur ausübt. Zugabe des BASE-Peptides führt zur Interaktion mit der ACID-Sequenz, wodurch beide Peptidsequenzen eine starre helicale coiled-coil Konformation annehmen. Wegen der beidseitigen Verknüpfung entsteht Spannung. Ein nur einseitig fixiertes ACID-Peptid sollte spannungsfrei mit zugesetztem BASE-Peptid, oder BASE-Peptid fusioniert an weitere Partner interagieren können. Auf jeden Fall bleibt die Partikelstruktur des ACID-Fusionsproteins im herkömmlichen kontinuierlichen core-System nach Spaltung der C-terminalen Verbindung zum Core-Träger mittels TEV-Protease erhalten.

Split-Cores mit exponierter ACID-Sequenz können mit Fremdproteinen beladen werden, an die das komplementäre BASE-Peptid gekoppelt ist. Dies ist eine von mehreren Möglichkeiten, split-Core CLPs nachträglich mit Fremdmolekülen der Wahl zu beladen.

Durch die oben näher erläuterten Kupplungsmechanismen ist es möglich, CLPs mit einem Teil der Kupplung bereitzustellen. Das an den anderen Teil der Kupplung fusionierte Protein kann leicht an die CLPs gekoppelt werden. Auf diese Art können beispielsweise nicht peptidartige Strukturen dem Immunsystem präsentiert werden.

### e) Simultane Fusion unterschiedlicher Fremdmoleküle an coreN und coreC

In den bisherigen Beispielen wurde jeweils *eine* Fremdsequenz an entweder coreN oder coreC fusioniert. Grundsätzlich können auch *zwei unterschiedliche* Fremdmoleküle simultan an coreN und coreC fusioniert werden; dies könnten bspw. die beiden Untereinheiten eines heterodimeren Fremdproteins sein. Als Modell wurde ein zweigeteiltes GFP benutzt, wovon das eine Segment, die ersten 10 der 11 β-Stränge von GFP enthaltend, an coreN und das zweite Segment, den 11. β-Strang von GFP enthaltend, an coreC fusioniert wurde. Ko-Expression von einem Typ1-Vektor führte zur Entstehung grün fluoreszierender CLPs. Somit hatten sich sowohl die coreN- und coreC-Domänen zu einer assemblierungsfähigen CLP-Struktur zusammengelagert wie auch die beiden GFP-Teile zu einer korrekten 3D-Struktur unter Ausbildung des GFP-Chromophors ergänzt.

Das Beispiel zeigt, dass die simultane Fusion unterschiedlicher Fremdsequenzen an coreN und coreC unter Ausbildung funktioneller Strukturen sowohl des split-Core-Trägers wie auch der beiden unterschiedlichen an coreN und coreC fusionierten Fremdsequenzen möglich ist.

### Beispiel 12

### Split-Core-CLPs, welche die GB1-Domäne von Protein G auf der Oberfläche präsentieren

Als weiteres konkretes Beispiel wurde die sog. GB1 Domäne aus dem mit Protein A funktionell verwandten *Streptococcus spp.* Immunglobulin-Bindeprotein "Protein G" in das HBV split-Core System eingeführt. Protein G besteht ebenfalls aus mehreren ähnlichen Domänen. Protein G bindet wie Protein A an Immunglobuline, hat jedoch andere Spezifitäten bez. Speziesherkunft und Subtypen der IgGs. Wegen dieser Komplementarität zwischen Protein G und Protein A werden beide als kommerzielle Reagenzien für immunologische Nachweise angeboten, die auf der Bindung an unterschiedlichen Immunglobuline beruhen. Wie Protein A bindet auch Protein G und die von ihm abgeleitete GB1-Domäne den konstanten (Fc) Teil des Antikörpers, der variable, Antigen-erkennende Teil des Antikörpers bleibt für die Bindung an das Antigen zugänglich. Somit kann eine Vielzahl unterschiedlicher Antikörper gebunden werden.

In diesem Beispiel wurde die GB1-Domäne in das split-core-System eingeführt und gezeigt, dass (i) CLPs gebildet werden und (ii), dass diese CLPs - aber nicht Kontroll-CLPs ohne GB1 - in der Lage sind, unterschiedliche Antikörper zu binden.

Analog den zuvor genannten Beispielen wurde die GB1-Domäne genetisch mit coreN bzw. mit coreC fusioniert und mit den jeweils fehlenden coreC bzw. coreN-Fragmenten koexprimiert.

Die spezifische AS-Sequenz entspricht den AS 229 bis 284 des Protein G Precursor-Proteins (Swiss Prot Accession: P06654); die 3D-Struktur ist bekannt (PDB Accession: 1 PGA; Ref: Gallagher T, Alexander P, Bryan P, Gilliland GL (1994) Two crystal structures of the B1 immunoglobulin-binding domain of streptococcal protein G and comparison with NMR Biochemistry 33: 4721-4729)
coreD78-*GGGGSGGGGTQ-*YKLILNGKTLKGETTTEAVDAATAEKVFKQYANDNGVDGEWTYDDATKTFTVTEdP

Die Verknüpfung an coreN erfolgte über einen *G4SG4TQ*-Linker; die vorletzte AS K wurde durch D (Kleinschreibung in der Sequenz) ersetzt; der vorhergehende E-Rest ist die letzte in der Röntgenstruktur sichtbare AS; durch den K>D-Austausch lässt sich auf Nukleotid-Ebene eine BamHI-Schnittstelle einführen (GAg gat cct TAG); diese befindet sich damit an homologer Position zu den vorbeschriebenen Konstrukten.

Wie in den anderen Beispielen kam es zur effizienten Bildung von Partikeln, nachgewiesen durch Sucrose-Gradienten-Sedimentation, Laufverhalten im nativen Agarosegel, und durch Elektronen-Mikroskopie.

Zum Nachweis der Interaktionsfähigkeit der CLP-getragenenen GB1-Domänen wurden die Partikel mit Immunglobulinen inkubiert, anschließend erneut im Sucrose-Gradienten sedimentiert; dabei kam es zur eindeutigen Ko-Sedimentation der Antikörper mit den Partikeln. Zusätzlich ließ sich die IgG-Bindung dadurch nachweisen, dass das coreN-GB1 Fragment auf Western Blots nach SDS-Gelelektrophorese direkt mit Sekundär-Antikörper-Konjugaten reagierte. Auch im Kontext der Fusion an coreN war daher das modifizierte GB1 in der Lage, effizient zu renaturieren und an seinen Liganden, IgG, zu binden.

Zum Nachweis, dass tatsächlich das Partikel-gebundene GB1 Immunglobulin bindet, wurde ein Aliquot der entsprechenden Sucrose-Gradientenfraktion mit einem mit 5 nm Goldmarkierten Antikörper inkubiert, ungebundener Antikörper durch erneute Sedimentation im Sucrosegradienten abgetrennt, und die Partikelfraktion elektronenmikroskopisch analysiert. Die elektronendichten Gold-Partikel zeigten die Bindung des Antikörpers an intakte CLPs.

### Beispiel 13

### Selbst-fluoreszierende GFP- und GFP-Varianten-haltige split-Core-CLPs, welche interaktionsfähige Fremdmoleküle präsentieren

Beispiel 11 e) erläutert Fusionen mit Bedeutung jenseits von Vakzine-Anwendungen, wobei ein in zwei Teilen vorliegendes GFP-Molekül (GFP ß1-10 und GFP ß11) unter Ausbildung der nativen GFP-Struktur (grüne Fluoreszenz) und unter Bildung von CLPs assoziiert, wenn GFPß1-10 an coreN, und GFPß11 an coreC fusioniert ist.

Im vorliegenden Beispiel wurden an GFPß11 zusätzliche Fremdsequenzen fusioniert, nämlich die GB1-Domäne aus Protein G bzw. die PräS-Domänen der Oberflächenproteine des humanen HBV, des Enten-HBV, und des Reiher-HBV. Dies ist nur möglich, weil durch die split-Anordnung ein neuer N-Terminus an GFPß11 entsteht, der zudem durch die spezifische Orientierung auf den split-Core-CLPs von der CLP-Oberfläche nach außen zeigt. Die Konstruktion der jeweiligen Expressionsplasmide erfolgte analog den zuvor beschriebenen Verfahren in split-Core-Vektoren, die für separate coreN und coreC-Fragmente kodieren, in diesem Fall bereits verknüpft mit GFPß1-10 an coreN, und GFPß11 an coreC.

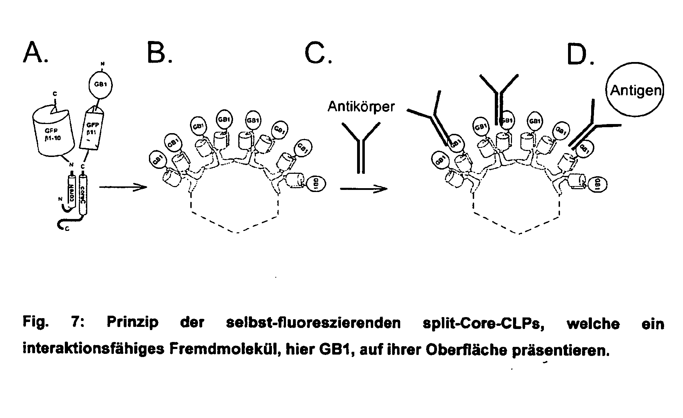

Anhand der bisherigen Daten war erwartet worden, dass der N-Terminus des GFPß11-Segments weg von der CLP-Oberfläche nach außen zeigen würde, und somit die Anwesenheit einer weiteren Fremddomäne nicht grundsätzlich mit der Fähigkeit der coreN und coreC-Anteile zur Bildung einer Partikel-Struktur, und nicht mit der Fähigkeit der GFPß1-10 und GFPß11-Anteile zur Bildung der nativen GFP-Struktur interferieren würde. Das Ergebnis dieses Beispiels ist in Fig. 7 schematisch dargestellt. In dieser Figur zeigen die Teilabbildungen A-D folgendes:
A. Grundstruktur der beiden Teile des Fusionsproteins. GFPß1-10 ist an coreN fusioniert, GFPß11 an coreC. Da GFPß11 einen neuen N-Terminus bereitstellt, kann an diesen eine weitere Domäne, hier GB1 fusioniert werden. B. Selbst-fluoresziererende CLPs entstehen durch Interaktion von coreN mit coreC (CLP-Bildung), und von GFPß1-10 mit GFPß11 (Bildung des GFP-Chromophors), wie bereits vorher gezeigt. Zusätzlich präsentieren diese jedoch GB1 in zugänglicher Form auf ihrer Oberfläche. C An die GB1-Domänen können unterschiedliche Antikörper über ihren Fc-Teil gebunden werden. D. Über ihre unterschiedlichen variablen Teile können die Antikörper ihrerseits mit ihrem spezifischen Antigen interagieren. Split-Core-GFP-GB1-CLPs können somit den üblichen Fluoreszenz-markierten Sekundärantikörper in der indirekten Immunfluoreszenz ersetzen. Eine Anwendung zur Fluoreszenz-Markierung von Tubulin in zwei unterschiedlichen Zellen ist in Abb. 7 gezeigt. Analog sind andere Anwendungen möglich, die auf der Bindung von Antikörper an Antigen beruhen.

Tatsächlich ließen sich die weiter modifizierten Fragmente effizient in E. coli ko-exprimieren und bildeten laut Sedimentation im Sucrose-Gradienten sowie Laufverhalten im nativen Agarosegel CLPs. Zusätzlich zeigten diese CLPs die typische GFP-Fluoreszenz, somit hatten sich auch die GFP-Anteile in korrekter Weise zusammengefunden. Das Beispiel zeigt, dass auch die GB1-Anteile korrekt gefaltet und auf der CLP-Oberfläche zugänglich vorlagen.

Zum Nachweis der Bindungsfähigkeit der selbstfluoreszierenden GB1-präsentierenden CLPs an Antigen-gebundene Antikörper wurden HeLa (aus Mensch) bzw. LMH-Zellen (aus Huhn) permeabilisiert, dann mit einem Maus-Erst-Antikörper gegen Tubulin inkubiert. In einem Fall wurden die Zellen konventionell mit einem chemisch Fluoreszenz-markierten (Cy2) Zweit-Antikörper (Ziege-anti-Maus), oder alternativ mit den selbst-fluoreszierenden, GB1-präsentierenden split-Core CLPs inkubiert, dann gewaschen; schließlich wurden die Zellen fluoreszenz-mikroskopisch observiert.

In beiden Fällen wurden vergleichbare Fluoreszenz-Bilder (wie erwartet, cytoplasmatische Anfärbung unter Aussparung des Zellkerns) erhalten. Somit müssen die GB1-Domänen zur Bindung an den an Tubulin gebundenen anti-Tubulin-Erst-Antikörper in der Lage gewesen sein.

Selbstfluoreszierende split-Core CLPs, welche GB1 präsentieren, können somit alternativ zu konventionellen Fluoreszenz-markierten Sekundär-Antikörper-Konjugaten zum Nachweis eines Antigen-gebundenen Erst-Antikörpers eingesetzt werden.

Dies belegt, dass die GB1-Domänen nach wie vor in der Lage sind, mit IgG zu interagieren, sie müssen daher korrekt gefaltet vorliegen. Zudem belegt es, dass das die selbstfluoreszierenden. GB1-präsentierenden CLPs als generelles Detektionsreagenz (vergleichbar einem fluoreszenz-markierten Sekundärantikörper) eingesetzt werden können.

Analoge Konstrukte wurden mit Farbvarianten von GFP erhalten, in welchen bekannte Mutationen, die das Absorptions- und Emissionsspektrum verändern, durch gerichtete Mutagenese in das vorhandene GFP-Konstrukt eingeführt wurden. Spezifisch wurden gelbfluoreszierende (Yellow fluorescent protein; YFP) und cyan-fluoreszierende (Cyan fluorescent protein; CFP) Varianten hergestellt. Beide bildeten CLPs, die die jeweils typischen Absorptionsspektren zeigten. Somit können GB1-präsentierende split-Core-GFP-CLPs mit unterschiedlichen Chromophor-Eigenschaften erzeugt werden, die für Mehrfach-Immunfluoreszenz-Anwendungen geeignet sind..

Das split-core System erlaubt die Herstellung selbst-fluoreszierender CLPs, welche auf ihrer Oberfläche ein interaktionsfähiges weiteres Fremdmolekül präsentieren. Ist dieses Fremdmolekül GB1, so ist es weiterhin in der Lage, mit Immunglobulinen spezifisch zu interagieren, auch wenn diese an ihr Ziel-Antigen gebunden sind. Hieraus ergibt sich eine neue, über Vakzine-Anwendungen hinausgehende Anwendungsmöglichkeit, nämlich der direkte Nachweis, via Fluoreszenz, von unterschiedlichen Antigenen mittels der selbstfluoreszierenden CLPs. Besonders geeignet sind solche selbstfluoreszierenden, interaktionsfähigen Split-Core-CLPs für diagnostische und analytische Anwendungen.

### Selbst-fluoreszierende split-Core-CLPs, welche andere Fremddomänen als GB1-aus Protein G präsentieren

Die o.g. Ergebnisse zeigen, dass auf split-Core-CLPs, welche zusätzlich ein zweigeteiltes GFP enthalten, eine weitere Fremddomäne, GB1, in funktioneller, d.h. mit dem spezifischen Liganden IgG interaktionsfähiger, Form präsentiert werden können. Allerdings ist GB1 mit weniger als 60 AS eine relativ kleine Domäne. Um die Generalisierbarkeit nachzuweisen, wurden homologe Konstrukte hergestellt, in denen GB1 durch die PräS-Domänen der großen Hüllproteine der HBVs von Mensch (HBV), Ente (DHBV) und Reiher (HHBV). Diese Domänen vermitteln die spezifische Bindung der jeweiligen Viren an ihre natürlichen Zielzellen, d.h. Hepatozyten der entsprechenden Wirtsspezies. Mit 108 AS für HBV, und ca. 160 AS für DHBV und HHBV PräS sind sie deutlich größer als GB1 und zudem in ihrer Sequenz von GB1, aber auch untereinander unterschiedlich.

Alle drei Domänen wurden analog GB1 in split-Core-GFP Fusionsproteine inseriert (vgl. Fig. 7; statt GB1 die entsprechenden PräS-Domänen). Alle 3 bildeten effizient CLPs, wie durch Sucrose-Gradienten Sedimentation, Laufverhalten in der nativen Agarose-Gelelektrophorese, sowie durch Elektronenmikroskopie nachgewiesen wurde; die CLPs zeigten die typische GFP-Fluoreszenz (d.h. die GFP-Anteile waren zur korrekten 3D-Struktur von GFP assoziiert); zudem reagierten die HBV-PräS- und DHBV-PräS-CLPs mit monoklonalen Antikörpern gegen HBV-PräS und DHBV-PräS; ein entsprechender Antikörper gegen das Reiher-Virus-PräS ist derzeit nicht verfügbar.

Eine weitere Anwendung ist das Aufspüren der zellulären Rezeptoren für die jeweiligen Viren. Es lässt sich annehmen, dass durch die große Anzahl (240) PräS-Domänen pro Partikel die CLPs eine enorm gesteigerte Avidität für die Rezeptoren besitzen, so dass diese stabil gebunden und so auch identifiziert werden können. Rezeptor-tragende Zellen würden via GFP-Fluoreszenz der CLPs mittels Fluoreszenz-Mikroskopie oder FACS identifiziert; solubilisierte Rezeptormoleküle aus solchen Zellen würden mit den CLPs inkubiert und sollten sich bei nachfolgender Sedimentation in Sucrose-Gradienten in den Partikeltypischen Gradienten-Fraktionen anreichern.

### Beispiel 14

### Split-CoreOspA CLPs mit OspA an coreN erzeugen höhere, mit OspA an coreC niedrigere Titer an protektiven anti-OspA Antikörpern als LipOspA

Wie in der Figur 5 gezeigt, lässt sich durch Verknüpfung an entweder coreN oder coreC die Orientierung eines präsentierten Fremdproteins gezielt beeinflussen. Im Fall von OspA wird dadurch entweder das C terminal gelegene Epitop des bekannt neutralisierenden monoklonalen Antikörpers LA-2 besonders gut exponiert (Fusion an coreN) oder stattdessen der N terminale OspA-Bereich, in dem keine bekannten neutralisierenden Epitope liegen (Fusion an coreC). Wie in der Figur 4 gezeigt, ergeben beide split-Core-OspA CLPs (dort Gruppe 2 für coreN-, und Gruppe 6 für coreC-Fusion) hohe Gesamt-anti-OspA Antikörper-Titer, der Anteil LA-2-äquivalenter Antikörper ist jedoch - wie aus der Struktur erwartet - bei Fusion an coreN wesentlich höher.

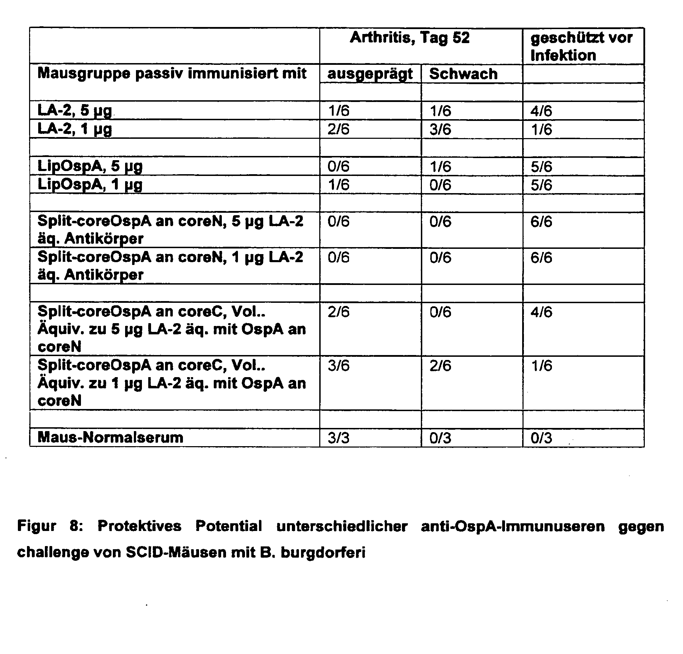

Allerdings handelte es sich hierbei um in vitro Daten. Um direkt das protektive Potential der induzierten Antikörper in vivo zu bestimmen, wurde die Fähigkeit der jeweiligen Immunseren zur Neutralisation einer artifiziell gesetzten *B. burgdorferi* Infektion in einem etablierten Mausmodell erfasst (vgl. Nassal et al., Eur. J. Immunol. 2005; S. 655-665). SCID-Mäuse wurden mit en entsprechenden Immunseron i.p inokuliert, 2 Studen später erfolgte s.c. *challenge* mit je 10³/Maus Spirochaeten. In ungeschützten Mäusen führt dies regelmäßig zur Infektion, die sich in Arthritis-artiger Symptomatik äußert, welche im linken und rechten Tibiotarsal-Gelenk semiquantitativ erfasst werden kann. Als Negativkontrolle diente eine Gruppe von 3 Mäusen, die lediglich Normal-Maus-Serum erhielten. Als Positivkontrolle dienten Gruppen von je 6 Mäusen, die entweder den gereinigten, bekannt neutralisierenden monoklonalen Antikörper LA2, oder mit LipOspA induzierte Antikörper erhielten. Die Testgruppen (ebenfalls je 6 Tiere) erhielten Immunseren, die durch split-CoreOspA mit OspA an coreN bzw. dito an coreC erhalten worden waren. Um quantitative Unterschiede zu erfassen, erfolgte die passive Immunisierung einmal mit 5 µg und einmal mit 1 µg LA2 bzw. der diesen LA2-Mengen äquivalenten Volumina an Immunseren von LipOspA bzw. split-Core-OspA mit OspA an coreN vakzinierten Tieren. Die Ergebnisse sind in Fig. 8 dargestellt.

Wegen des geringen Anteils an LA2-äquivalenten Antikörpern in den mittels split-Core-OspA mit OspA an coreC erhaltenen Immunseren wurden hiervon äquivalente Volumina wie von split-Core-OspA mit OspA an coreN eingesetzt, ohne Rücksicht auf den LA2 äquivalenten Titer. Arthritis scores wurden an Tag 13, 17, 21, 28, 35, 45 und 52 bestimmt. Die Ergebnisse für Tag 52 in Figur 8 zusammengefasst.

Alle mit Normal-Maus-Serum inokulierten Tiere entwickelten, wie erwartet, Arthritis. Die Protektion durch den monoklonalen Antikörper LA-2 verringerte sich bei Dosis-Reduzierung von 5 auf 1 µg von 4/6 auf 1/6 Tiere, durch die LipOspA Immunseren wurden 5/6 Tieren geschützt. Passive Immunisierung mit via split-CoreOspA mit OspA an coreN erhaltenen Immunseren schützte alle (6/6) Tiere, auch bei der geringen Dosierung, vor Infektion. OspA an coreC hatte eine deutlich geringere protektive Aktivität (4/6 Tieren bei der hohen, 1/6 Tieren bei der niedrigen Dosierung).

Split-Core-OspA CLPs mit OspA an coreN führen zu hohen Titern an LA2-äquivalenten Antikörpern und diese sind in vivo protektiv. Das protektive Potential übersteigt dasjenige von LipOspA-induzierten Antikörpern. Split-Core-OspA-CLPs mit OspA an coreC induzieren hohe Gesamttiter an anti-OspA-Antikörpern, diese sind jedoch nur zu einem geringen Anteil LA-2-äquivalent und besitzen nur ein geringeres neutralisierendes Potential. Fusion eines Fremdantigens an coreN bewirkt somit eine qualitativ distinkte Immunantwort von der Fusion an coreC.

### Beispiel 15

### Cirumsporozoiten-Protein (CSP) des Malaria-Erregers Plasmodium falciparum

Voll-Längen CSP im split-Core-System induziert eine stärkere und breitere Immunantwort als die immundominante CSP-repeat-Sequenz im konventionellen, kontinuierlichen Core-System

Bisher war gezeigt worden, dass sich das komplette CSP mit einer Länge von 319 AS ausschließlich im split-Core-System unter CLP-Bildung exprimieren ließ; im konventionellen kontinuierlichen System war dies nicht möglich. Eine verkürzte Version ohne die Cysteinreiche C terminal Domäne (nachfolgend CSPshort) ließ sich zwar im konventionellen kontinuierlichen System auch darstellen, das Protein zeigte jedoch nach wenigen Tagen eine ausgeprägte Präzipitationstendenz - im split-Core-System trat diese nicht auf, was überlegene protein-chemische Eigenschaften belegt.

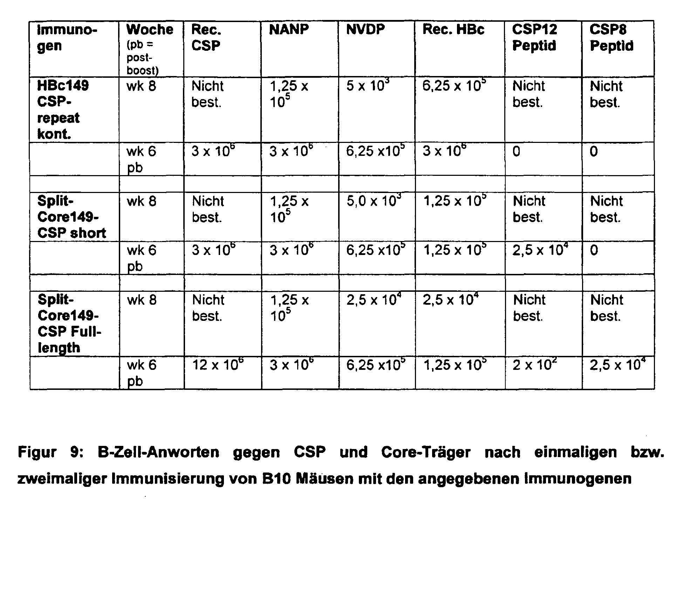

Eine Besonderheit des CSP ist die Anwesenheit einer mehrfachen Wiederholung der Tetrapeptid-Motive NANP und NVDP (in der hier benutzten Vollängen-Version 24 NANP und 3 NVDP-Motive); diese sind bekannt immunogen und eine experimentelle Vakzine basierend auf dem konventionellen kontinuierlichen Core-System ist beschrieben. Für eine vergleichende Immunogenitätsstudie wurde daher ein entsprechendes konventionelles Konstrukt mit der repeat-Sequenz NANPNVDP(NANP)₃NVDP zwischen Core-AS D78 und S81 hergestellt, das - wie erwartet, da die Insertion klein ist - CLPs bildete. Dieses diente in einer Immunogenitätsstudie als Vergleich für Vollängen-CSP und das C terminal verkürzte CSPshort. Mäuse wurden mit jeweils gleichen Mengen der jeweiligen CLPs immunisiert (20 µg/Maus); nach 2, 4, 6 und 8 Wochen wurden die gebildeten Antikörper-Mengen per ELISA bestimmt; dann erfolgte eine boost-Immunisierung (analog der ersten), und die Titer wurden 2, 4, und 6 Wochen nach boost erneut bestimmt. Als Testantigene dienten rekombinantes CSP (als Fusionprotein aus E. coli), NANP und NVDP-Peptide, sowie rekombinantes HBcAg, um die B-Zellantwort gegen den Träger zu bestimmen. Die Antigene wurden auf ELISA-Platten immobilisiert, dann mit 2-fachen Verdünnungsreihen der Immunseren getestet. Die größtmögliche Verdünnung, die ein über dem Hintergrund liegendes Signal ergab, ist in der Figur 9 angegeben.

### Hieraus ergeben sich die folgenden Schlussfolgerungen:

### a) B-Zell-Antwort gegen rekombinantes CSP

Alle drei Immunogene ergeben eine äußerst starke Antwort gegen CSP; für Voll-Längen-CSP liegt diese jedoch noch 4-fach höher (Titer: 1 : 12 x 10⁶ vs. 1 : 3 x 10⁶ für repeat bzw. verkürztes CSP); dies bedeutet, dass eine 12-Millionen-fache Verdünnung (!) des Immunserums noch in der Lage ist, CSP Protein zu erkennen.

### b) B-Zell-Antworten gegen repeat-Peptide NANP und NVDP

Die Antworten gegen das NANP-Peptid sind erwartungsgemäß für alle Konstrukte ähnlich; Die Antworten gegen das weniger oft im repeat vorhandene NVDP-Peptid sind ähnlich, beim Voll-Längen-CSP-Konstrukt jedoch schon nach der ersten Immunisierung (ohne boost) deutlich höher (Titer 1 : 2.5 x 10⁴ vs. 1 : 5 x 10³).

### c) B-Zell-Antworten gegen nicht im repeat enthaltene CSP-Sequenzen

Ausschließlich CSPshort und Voll-Längen-CSP induzieren eine Antwort gegen die Peptide CSP12 (EEPSDKHIEQYLKKIQNSLS; Pos. 246-264 im Voll-Längen split-Core-Konstrukt) und CSP8 (GNGIQVRIKPGSANKPKDELD; Pos. 274-294 im Voll-Längen split-Core-Konstrukt). Das Fehlen entsprechender Antworten nach Immunisierung mit dem repeat-Peptid im kontinuierlichem Core-System ist erwartet, da dort die CSP8 und CSP12 Sequenzen nicht enthalten sind. Die Bedeutung des Ergebnisses liegt darin, dass insbesondere das Voll-Längen-CSP ausschließlich im split-Core-System hergestellt werden kann; Mit dem split-Core-System können also zusätzliche Epitope angesprochen werden, die zur Schutzwirkung einer Impfung beitragen können.

### d) B-Zell-Antworten gegen den Core-Träger

Sowohl CSPshort wie auch Voll-Längen-CSP im split-core-System führen zu einer ca. 25-fach geringeren Antwort gegen den Core-Träger als das repeat-Peptid im kontinuierlichen System (Titer 1 : 1,25 x 10⁵ vs. 3 x 10⁶). Da die erwünschte Antwort gegen das Fremdprotein mindestens genauso hoch (CSPshort) oder noch höher (Voll-Längen-CSP) ist wie bei Verwendung des repeat-Peptides im kontinuierlichen Core-System, ist die spezifische Immunogenität bez. der inserierten Fremdsequenz bei Verwendung des verkürzten, und besonders des Voll-Längen-CSP im split-Core-System wesentlich verbessert. Hinzu kommt, dass eine starke Antwort gegen den Core-Träger die Differenzial-Diagnostik von anti-HBsAgpositiven Personen (Infektion vs. anti-HBsAg durch Vakzinierung) erschwert; eine geringe anti-Core-Träger-Antwort ist daher erwünscht.

### e) Voll-Längen-CSP im split-Core-System aktiviert T.Zellen

Zum Nachweis der Induktion einer T-Zell-Antwort wurden Milzzellen der jeweilig immunisierten Mäuse mit rec. HBcAg bzw. einem bekannten T-Zell-Peptid (HBc 120-140) oder mit rec. CSP bzw. den CSP-Peptiden NANP und CSP8 stimuliert, dann die IL-2-Produktion gemessen. Alle Konstrukte führten zum Auftreten von T-Zellen, die durch HBcAg, HBc120-140, rec. CSP und NANP aktivierbar waren. Ausschließlich Milzzellen von mit Voll-Längen-CSP im split-core-System immunisierten Mäusen waren jedoch durch das CSP8 Peptid zur IL-2-Produktion anzuregen.

Auch auf T-Zell-Ebene führt daher das Voll-Längen-CSP Konstrukt zu einer breiteren Antwort als das kontinuierliche Nur-repeat-Peptid-Konstrukt.

## Patentansprüche

1. Split-Core-Trägersystem, das als getrennte Polypeptide die Core N- und die Core C-Domäne des Core-Proteins eines Hepatitis B-Virus und wenigstens ein Fremdmolekül auf Peptidbasis, gegen das eine Immunantwort hervorgerufen werden sollen, aufweist, wobei das Fremdmolekül an den C-Terminus der Core N-Domäne oder an den N-Terminus der Core C-Domäne fusioniert ist und das Core-Protein capsid-like-particles bilden kann und wobei das Fremdmolekül an eine Aminosäure des Core-Proteins fusioniert ist, die zwischen der Position 73 und 94 des Core-Proteins liegt.

2. Split-Core-Trägersystem nach Anspruch 1, **dadurch gekennzeichnet, dass** das Hepatitis B-Virus für Säugetiere spezifisch ist.

3. Split-Core-Trägersystem nach Anspruch 2, **dadurch gekennzeichnet, dass** das Core-Protein die Sequenz eines humanen Hepatitis B-Virus aufweist.

4. Split-Core-Trägersystem nach Anspruch 2, **dadurch gekennzeichnet, dass** das Core-Protein die Aminosäuresequenz des HBV des Nordamerikanischen Waldmurmeltieres aufweist.

5. Split-Core-Trägersystem nach einem der Ansprüche 1 bis 4, **dadurch gekennzeichnet, dass** das Fremdmolekül eine Aminosäuresequenz eines Proteins eines pathogenen Bakteriums ist.

6. Split-Core-Trägersystem nach einem der Ansprüche 1 bis 4, **dadurch gekennzeichnet, dass** das Fremdmolekül eine Aminosäuresequenz eines Proteins eines pathogenen Eukaryonten, insbesondere des Malaria-Erregers Plasmodium falciparum ist.

7. Split-Core-Trägersystem nach einem der Ansprüche 1 bis 4, **dadurch gekennzeichnet, dass** das Fremdmolekül eine Aminosäuresequenz eines Proteins eines Virus ist.

8. Split-Core-Trägersystem nach einem der Ansprüche 1 bis 4, **dadurch gekennzeichnet, dass** das Fremdmolekül eine Aminosäuresequenz eines Proteins mit eigenem Pathogenitäts-bezogenen Potential, insbesondere ein Tumormarkerprotein, ist.

9. Verfahren zur Herstellung eines Trägers für Fremdproteine, gegen die eine Immunantwort induziert werden soll, der einerseits eine Proteinsequenz des Core-Antigens eines Hepatitis B-Virus aufweist und andererseits eine heterologe Proteinsequenz als Fremdmolekül,
wobei die heterologe Proteinsequenz nach der Position 73 und vor der Position 94 der Hepatitis B-Coreregion an die Core N oder die Core C Domäne anfusioniert wird und,
wobei die Polypeptidkette des Trägers entweder nach der Domäne Core N oder vor der Domäne Core C unterbrochen wird, **dadurch gekennzeichnet, dass**
entweder die Domäne Core N und die heterologe Proteinsequenz oder die Domäne Core C und die heterologe Proteinsequenz separat als Fusionsproteine exprimiert werden
oder, dass zwischen Core N und heterologer Proteinsequenz oder zwischen heterologer Proteinsequenz und Core C eine Erkennungssequenz für eine Protease eingefügt wird und der Split-Core-Impfstoff nach der Expression, aber vor der Verwendung mit der Protease in zwei Polypeptide gespalten wird, und,
dass capsid-like particles gebildet werden.

10. Verfahren nach Anspruch 9, **dadurch gekennzeichnet, dass** die heterologe Proteinsequenz nach der Aminosäure 74 und or die Aminosäure 85 des Core-Antigens von Hepatitis B anfusioniert wird.

11. Verfahren nach einem der Ansprüche 9-10, **dadurch gekennzeichnet, dass** die heterologe Fremdproteinsequenz wenigstens 40 Aminosäuren lang ist.

12. Verfahren nach Anspruch 9, **dadurch gekennzeichnet, dass** die heterologe Fremdproteinsequenz wenigstens 120 Aminosäuren lang ist.

13. Verfahren nach einem der Ansprüche 9-11, **dadurch gekennzeichnet, dass** zwei separate Polypeptide mit Hilfe eines bicistronischen Vektors exprimiert werden, wobei das eine Polypeptid die Core N-Domäne und das andere Polypeptid die Core C-Domäne beinhaltet und die heterologe Proteinsequenz entweder mit dem C-Terminus der Core N-Domäne oder mit dem N-Terminus der Core C-Domäne fusioniert wird.

14. Arzneimittel, das Capsid-ähnliche Partikel aufweist, **dadurch gekennzeichnet, dass** diese Partikel ein Split-Core-Trägersystem nach einem der Ansprüche 1 bis 8 aufweisen.

15. Arzneimittel nach Anspruch 14, **dadurch gekennzeichnet, dass** es sich um einen Impfstoff handelt.

16. Diagnostisches Mittel, das Capsid-ähnliche Partikel aufweist, **dadurch gekennzeichnet, dass** diese Partikel ein Split-Core-Trägersystem nach einem der Ansprüche 1 bis 8 aufweisen.

## Claims

1. A split-core carrier system which has, as separate polypeptides, the core N domain and the core C domain of the core protein of a hepatitis B virus and at least one foreign molecule on the basis of peptides to which an immune response is to be elicited, said foreign molecule being fused to the C terminus of the core N domain or to the N terminus of the core C domain and the core protein being able to form capsid-like particles, and the foreign molecule being fused to a core protein amino acid which is located between positions 73 and 94 of the core protein.

2. The split-core carrier system as claimed in claim 1, **characterized in that** the hepatitis B virus is specific to mammals.

3. The split-core carrier system as claimed in claim 2, **characterized in that** the core protein has the sequence of a human hepatitis B virus.

4. The split-core carrier system as claimed in claim 2, **characterized in that** the core protein has the amino acid sequence of the WHV of the northern American woodchuck.

5. The split-core carrier system as claimed in any of claims 1 to 4, **characterized in that** the foreign molecule is an amino acid sequence of a protein of a pathogenic bacterium.

6. The split-core carrier system as claimed in any of claims 1 to 4, **characterized in that** the foreign molecule is an amino acid sequence of a protein of a pathogenic eukaryote, in particular of the malaria pathogen *Plasmodium falciparum.*

7. The split-core carrier system as claimed in any of claims 1 to 4, **characterized in that** the foreign molecule is an amino acid sequence of a protein of a virus.

8. The split-core carrier system as claimed in any of claims 1 to 4, **characterized in that** the foreign molecule is an amino acid sequence of a protein having its own pathogenicity-based potential, in particular a tumor marker protein.

9. A method of preparing a carrier for foreign proteins, to which an immune response is to be induced, which carrier firstly has a protein sequence of the core antigen of a hepatitis B virus and secondly has as foreign molecule a heterologous protein sequence,
wherein the heterologous protein sequence is fused to the core N domain and the core C domain downstream of position 73 or upstream of position 94 of the hepatitis B core region, and
wherein the polypeptide chain of the carrier is interrupted either downstream of the core N domain or upstream of the core C domain, **characterized in that** either the core N domain and the heterologous protein sequence, or the core C domain and the heterologous protein sequence, are expressed separately as fusion proteins,
or that a recognition sequence for a protease is inserted between core N and heterologous protein sequence, or between heterologous protein sequence and core C, and the split-core vaccine is cleaved by the protease into two polypeptides after expression but before usage, and
that capsid-like particles are formed.

10. The method as claimed in claim 9, **characterized in that** the heterologous protein sequence is attached by fusion downstream of amino acid 74 and upstream of amino acid 85 of the core antigen of hepatitis B.

11. The method as claimed in either of claims 9-10, **characterized in that** the heterologous foreign protein sequence is at least 40 amino acids in length.

12. The method as claimed in claim 9, **characterized in that** the heterologous foreign protein sequence is at least 120 amino acids in length.

13. The method as claimed in any of claims 9-11, **characterized in that** two separate polypeptides are expressed with the aid of a bicistronic vector, the first polypeptide comprising the core N domain and the second polypeptide comprising the core C domain, and the heterologous protein sequence being fused either to the C terminus of the core N domain or to the N terminus of the core C domain.

14. A medicament having capsid-like particles, **characterized in that** said particles have a split-core carrier system as claimed in any of claims 1 to 8.

15. The medicament as claimed in claim 14, **characterized in that** it is a vaccine.

16. A diagnostic agent having capsid-like particles, **characterized in that** said particles have a split-core carrier system as claimed in any of claims 1 to 8.

## Revendications

1. Système porteur de la nucléocapside scindée qui présente, en tant que polypeptide séparé, les domaines nucléocapsidiques N-terminaux et C-terminaux de la protéine nucléocapsidique d'un virus de l'hépatite B et au moins une molécule étrangère à base de peptide, contre laquelle une réponse immunitaire devrait être suscitée, la molécule étrangère étant fusionnée au terminus C des domaines nucléocapsidiques N-terminaux ou au terminus N des domaines nucléocapsidiques C-terminaux et la protéine nucléocapsidique pouvant former des particules capsidiques et la molécule étrangère étant fusionnée à un acide aminé de la protéine nucléocapsidique qui est situé entre les positions 73 et 94 de la protéine nucléocapsidique.

2. Système porteur de la nucléocapside scindée selon la revendication 1, **caractérisé en ce que** le virus de l'hépatite B est spécifique des mammifères.

3. Système porteur de la nucléocapside scindée selon la revendication 2, **caractérisé en ce que** la protéine nucléocapsidique présente la séquence d'un virus de l'hépatite B humain.

4. Système porteur de la nucléocapside scindée selon la revendication 3, **caractérisé en ce que** la protéine nucléocapsidique présente la séquence d'acides aminés du VHB de la marmotte commune.

5. Système porteur de la nucléocapside scindée selon l'une des revendications 1 à 4, **caractérisé en ce que** la molécule étrangère est une séquence d'acides aminés d'une protéine d'une bactérie pathogène.

6. Système porteur de la nucléocapside scindée selon l'une des revendications 1 à 4, **caractérisé en ce que** la molécule étrangère est une séquence d'acides aminés d'une protéine d'un eucaryote pathogène, en particulier de l'agent pathogène du paludisme, Plasmodium falciparum.

7. Système porteur de la nucléocapside scindée selon l'une des revendications 1 à 4, **caractérisé en ce que** la molécule étrangère est une séquence d'acides aminés d'une protéine d'un virus.

8. Système porteur de la nucléocapside scindée selon l'une des revendications 1 à 4, **caractérisé en ce que** la molécule étrangère est une séquence d'acides aminés d'une protéine avec un potentiel propre lié au caractère pathogène, en particulier une protéine marqueuse de tumeur.

9. Procédé pour la production d'un support pour protéines étrangères, contre lesquelles une réponse immunitaire devrait être induite, qui, d'une part, présente une séquence protéique de l'antigène nucléocapsidique d'un virus de l'hépatite B et d'autre part, une séquence protéique hétérologue en tant que molécule étrangère,
la séquence protéique hétérologue étant fusionnée après la position 73 et avant la position 94 de la région nucléocapsidique de l'hépatite B aux domaines nucléocapsidiques N-terminaux ou nucléocapsidiques C-terminaux et,
la chaîne polypeptidique du support étant interrompue soit après les domaines nucléocapsidiques N-terminaux ou avant les domaines nucléocapsidiques C-terminaux, **caractérisé en ce que**
soit les domaines nucléocapsidiques N-terminaux et la séquence protéique hétérologue ou les domaines nucléocapsidiques C-terminaux et la séquence protéique hétérologue sont exprimés séparément comme protéines de fusion,
ou **en ce que**, entre la nucléocapside N-terminale et la séquence protéique hétérologue ou entre la séquence protéique hétérologue et la nucléocapside C-terminale, une séquence d'identification pour une protéase est insérée et le vaccin nucléocapsidique scindé est scindé après l'expression, mais avant l'utilisation avec la protéase en deux polypeptides, et **en ce que**
des particules capsidiques sont formées.

10. Procédé selon la revendication 9, **caractérisé en ce que** la séquence protéique hétérologue est fusionnée après l'acide aminé 74 et avant l'acide aminé 85 de l'antigène nucléocapsidique de l'hépatite B.

11. Procédé selon l'une des revendications 9 ou 10, **caractérisé en ce que** la séquence protéique étrangère hétérologue est longue d'au moins 40 acides aminés.

12. Procédé selon la revendication 9, **caractérisé en ce que** la séquence protéique étrangère hétérologue est longue d'au moins 120 acides aminés.

13. Procédé selon l'une des revendications 9 à 11, **caractérisé en ce que** deux polypeptides séparés sont exprimés avec l'aide d'un vecteur bicistronique, le un peptide contenant les domaines nucléocapsidiques N-terminaux et l'autre peptide les domaines nucléocapsidiques C-terminaux, la séquence protéique hétérologue étant fusionnée soit avec le terminus C des domaines nucléocapsidiques N-terminaux ou avec le terminus N des domaines nucléocapsidiques C-terminaux.

14. Médicament qui présente des particules capsidiques, **caractérisé en ce que** ces particules présentent un système porteur de la nucléocapside scindée selon l'une des revendications 1 à 8.

15. Médicament selon la revendication 14, **caractérisé en ce qu'**il s'agit d'un vaccin.

16. Moyen diagnostique qui présente des particules capsidiques, **caractérisé en ce que** ces particules présentent un système porteur de la nucléocapside scindée selon l'une des revendications 1 à 8.
